# EUROPÄISCHE PATENTSCHRIFT

(11) **EP 2 777 687 B1**
(45) Veröffentlichungstag und Bekanntmachung des Hinweises auf die Patenterteilung: **07.11.2018**
(21) Anmeldenummer: 13159634.8
(22) Anmeldetag: 15.03.2013
(51) Int. Cl.: A61K 6/00

(54) **Dentale Primerformulierung**
Dental primer formulation
Formulation de primaire dentaire

(43) Veröffentlichungstag der Anmeldung: 17.09.2014
(73) Patentinhaber: Ivoclar Vivadent AG, 9494 Schaan (LI)
(72) Erfinder: Catel, Dr. Delphine, 9470 Buchs SG (CH); Bock, Dr. Thorsten, 6800 Feldkirch (AT); Salz, Dr. Ulrich, 88131 Lindau (DE)
(74) Vertreter: Uexküll & Stolberg

(56) Entgegenhaltungen:
- US-A1- 2010 240 796
- RUSSELL D A ET AL: "Shear bond strength of resin composite to Dicor treated with 4-META.", THE INTERNATIONAL JOURNAL OF PROSTHODONTICS 1994 JAN-FEB, Bd. 7, Nr. 1, Januar 1994 (1994-01), Seiten 7-12, XP008164283, ISSN: 0893-2174

## Beschreibung

Die vorliegende Erfindung betrifft dentale Primerzusammensetzungen, die sich insbesondere zur Behandlung keramischer Oberflächen eignen.

In der Dentaltechnik unterscheidet man zwischen keramischen und metallischen Restaurationsmaterialien, wobei die keramischen Materialien weiter in silikatische Keramiken, wie z.B. Feldspat, Quarz, Leucit-basierte Keramiken und Glaskeramiken sowie Lithumdisilikat-basierte Keramiken ("Silikate"), und nichtsilikatische Keramiken, wie z.B. Yttrium-stabilisiertes Zirkonoxid, Aluminiumoxid, glasinfiltriertes Aluminiumoxid (z.B. In-CERAM Alumina, Fa. Vita, Deutschland), ("Oxidkeramiken") unterteilt werden. Bei den Metallen wird zwischen Nichtedelmetallen, wie z.B. Chrom, Nickel, Molybdän, Titan und deren Legierungen ("NEM"), und Edelmetallen, wie z.B. Gold, Platin, Palladium, Silber und deren Legierungen ("EM"), unterschieden.

Bei der Herstellung von Dentalrestaurationen ist es häufig erforderlich, diese Werkstoffe dauerhaft miteinander oder mit der natürlichen Zahnsubstanz zu verbinden. Hierzu werden in der Regel polymerisierbare Komposite und Zemente eingesetzt. Der Verbund wird nach dem Stand der Technik in einem zweistufigen Verfahren hergestellt. Im ersten Schritt wird die Oberfläche der Restaurationsmaterialien durch nasschemische oder mechanisch-abrasive Verfahren angeraut. Die Anrauhung dient der Schaffung einer mikroretentiven Oberflächenstruktur sowie der Oberflächenvergrößerung. Beide Aspekte sind dem Fachmann als wichtig für die Bereitstellung eines dauerhaft belastbaren Verbundes bekannt.

Das dentaltechnisch gebräuchliche Verfahren zur nasschemischen Erosion ("Ätzen") von Silikaten setzt vornehmlich wässrige Flusssäurelösungen ein. Trotz der positiven Effekte bezüglich der Verbundwirkung ist Flusssäure (HF) als Ätzmittel aus verschiedenen Gründen nachteilig. Zum einen ist in der dentalmedizinischer Anwendung der Einsatz von HF auf Grund der Toxizität mit einem Gefahrenpotential verbunden (G. Alex, Functional Esthetics & Restorative Dentistry, Series 2, Number 1, 38-49; Vohra et al., Clinical Toxicology (2008) 46, 79-84).

Neben ihrer nachteiligen Toxizität können HF-Lösungen zudem durch zu langes Einwirken die Keramik strukturell schädigen ("überätzen"), was sowohl die mechanischen Eigenschaften der Keramik, als auch den Verbund zum Befestigungszement verschlechtert (Barghi et al., Journal of Esthetic Restorative Dentistry (2006) 18, 47-52; Amaral et al., Brazilian Dental Journal (2011) 22, 245-248; Nagayassu et al., Brazilian Dental Journal (2006) 17, 290-295). Die Autoren der genannten Artikel kommen zum Schluss, dass die HF-Konzentration und HF-Einwirkdauer sorgfältig auf die zu ätzende Silikatkeramik abgestimmt sein müssen, um eine retentive Oberfläche zu schaffen ohne dabei die Keramik strukturell zu schädigen oder den Verbund zu schwächen.

Zur Vermeidung dieser Nachteile wurden in der Vergangenheit Bemühungen unternommen, Flusssäure durch weniger giftige Alternativstoffe zu ersetzen. Tylka et al, The Journal of Prosthetic Dentistry (1994) 72, 121-127, schlagen die Verwendung saurer Fluorphosphatlösungen zur intraoralen Anwendung vor. Della Bona et al., Int J Prosthodont (2002) 15, 159-167, vergleichen Ammoniumbifluorid (ABF), Fluorwasserstoffsäure und saure Fluorphosphatlösungen miteinander. HF ergab das deutlichste Ätzmuster, und es konnte gezeigt werden, dass HF zu einer signifikant besseren Haftfestigkeit als die anderen Materialien führt (Della Bona, Int J Prosthodont (2002) 15, 248-253). Cömlekoglu et al., Journal of Adhesive Dentistry (2009), 11, 447-453, fanden, dass die Behandlung von Keramikoberflächen mit wässriger Titantetrafluorid (TiF₄) Lösung die Ausbildung oberflächlicher TiO₂-Schichten bewirkt, die gute Haftwerte gewährleisten sollen. Die Autoren weisen aber darauf hin, dass die Haftung der TiO₂-Schichten an der Keramik durch Alterung beeinträchtigt werden kann. Die genannten HF-freien Verfahren sind bisher nicht zufriedenstellend.

Allen Ätzverfahren gemein ist, dass zur Vorbereitung einer adhäsiven Befestigung die silikatkeramischen Werkstücke mit der Ätzlösung kontaktiert werden und die Lösung nach einer gewissen Einwirkdauer mit Wasser abgespült wird. Im Anschluss ist die Silikatoberfläche mit Druckluft zu trocknen bevor weitere Arbeitsschritte ausgeführt werden können. Da bei der Reaktion zwischen Silikat und Ätzlösung bevorzugt die amorphen Bestandteile der Silikate zu löslichem Hexafluorsilikat umgewandelt werden und die kristallinen Bestandteile zurückbleiben, entsteht eine verbundfördernde rauhe (mikroretentive) Oberflächenstruktur.

Das gängigste mechanisch-abrasive Verfahren ist das Sandstrahlen, bei dem mit Druckluft beschleunigte Abrasionspartikel auf die zu behandelnde Oberfläche gerichtet werden. Durch die hohe kinetische Energie der Partikel wird eine mikroretentive Struktur erzielt. Wo der Einsatz des Sandstrahlens zu Beeinträchtigungen des Patienten führen kann (z.B. intraoral) oder geeignete Instrumente nicht zur Verfügung stehen, kann die Anrauhung z.B. auch durch rotierende Instrumenten herbeigeführt werden. Das Sandstrahlen ist für sichtbare Bereiche dentaler Restaurationen aus optischen Gründen nur bedingt geeignet.

Im zweiten Schritt wird die angeraute Restaurationsoberfläche durch einen Haftvermittler ("Primer") mit polymerisationsfähigen Funktionalitäten versehen ("primen"). Der Primer enthält Monomere, die einerseits über reaktive Gruppen an die Oberfläche der Restauration anbinden können und die andererseits über polymerisierbare Gruppen verfügen, welche die Copolymerisation mit weiteren Monomeren ermöglichen. Beispielsweise können Silane mit Silikatoberflächen unter Bildung kovalenter Si-O-Si-Bindungen reagieren, während Phosphate mit Zirkonoxid Zirkonium(oxophosphate) bilden. Die polymerisierbaren Gruppen können zu einem späteren Zeitpunkt mit dem zur Befestigung der Restauration eingesetzten Komposit oder Zement durch geeignete Verfahren copolymerisiert werden. Auf diese Weise wird ein dauerhafter, durch kovalente oder ionischen Bindungen gekennzeichneter Verbund zwischen Restauration und Komposit geschaffen.

Bislang bekannte Primer zur adhäsiven Befestigung dentaler Restaurationsmaterialien sind nicht in der Lage, Silikate zu ätzen, und eignen sich ausschließlich für die Oberflächenfunktionalisierung. Ein allein durch Silanisierung herbeigeführte Haftverbund ist jedoch deutlich schwächer, als der durch eine Kombination von HF-Ätzung und Silanisierung erzielte (Queiroz et al, Gen Dent. (2012), 60(2), 9-85). Für einen klinisch relevanten Verbund zu Silikaten ist damit nach aktuellem Stand zwingend eine vorherige Anrauhung der Oberfläche und die anschließende Silanisierung der angerauten Oberfläche in separaten Arbeitsschritten durchzuführen. Typische Primer des obigen Typs werden in den folgenden Dokumenten beschrieben.

EP 0 224 319 A1 beschreibt eine Primer-Zusammensetzung zur Haftverbesserung auf verschiedenen keramischen Materialien, die ein zu einem organofunktionellen Silanol hydrolysierbares Silan enthält.

Gegenstand von JP 2601254 B2 ist ein dentaler Primer für Keramiken und Metall, der die Kombination eines organofunktionellen Silans mit speziellen (meth)acryloylfunktionellen Phosphorsäuremonoestern enthält.

JP 2593850 B2 beschreibt eine adhäsive Dentalzusammensetzung, die u.a. ein organofunktionelles Silan und eine saure organische Phosphorverbindung mit einer radikalisch polymerisierbaren Doppelbindung enthält. Die Zusammensetzung soll die Bindung sowohl an Metalle als auch Keramik ermöglichen.

In DE 10 2005 002 750 A1 ist ein Primer für dentale Edelmetalllegierungen offenbart, der spezielle mit polymerisierbaren Gruppen substituierte Disulfide enthält.

Die EP 2 229 930 A1 offenbart eine dentale Haftvermittlerzusammensetzung, die sich für unterschiedliche Metalle und Keramiken eignet. Die Zusammensetzungen enthalten neben einen Alkoxysilan ein Phosphorsäureester-Monomer, ein schwefelhaltiges Monomer und Lösungsmittel.

Russell et al. (Int J Prosthodont 1994; 7: 7-12) beschreiben die Behandlung von Keramik-Oberflächen mit einer Kombination aus Dicor Etching Gel und Dicor Coupling Agent; das erste enthält Ammoniumhydrogendifluorid, das zweite ein polymerisierbares Alkoxysilan.

Primer, die zum simultanen Ätzen und Primen silikatischer Restaurationsoberflächen geeignet wären, werden im Stand der Technik nicht beschrieben.

Das separate Ätzen und Primen einer silikatkeramischen Restauration vor der adhäsiven Zementierung erfordert vom Behandler zahlreiche Arbeitsschritte, nämlich das Auftragen und Einwirken lassen eines Ätzgels auf die Silikatkeramik, das Abspülen des Ätzgels, das Trocknen der Oberfläche, das Auftragen und Einwirken lassen eines Primers und schließlich das Verblasen des Primers mit einem Luftbläser. Es handelt sich damit um ein sehr zeit- und arbeitsintensives Verfahren.

Der Erfindung liegt die Aufgabe zugrunde, einen dentalen Primer zur Verfügung zu stellen, der die oben genannten Nachteile nicht aufweist. Insbesondere soll der Primer ohne vorheriges Ätzen oder Anrauhen direkt auf die zu behandelnde Oberfläche aufgebracht werden können und einen sicheren Verbund unter oralen Bedingungen gewährleisten. Der Primer soll eine geringe Toxizität und eine hohe Stabilität aufweisen.

Erfindungsgemäß wird diese Aufgabe durch eine Primerzusammensetzung gelöst, welche die folgenden Komponenten enthält:
(1) mindestens ein Alkoxysilan-Monomer der allgemeinen Formel (I)

   R¹ₙSi(OR²)₄₋ₙ (I),

   worin
   - R¹: für einen organischen Rest steht, der mindestens eine polymerisierbare Gruppe aufweist,
   - R²: für H oder einen C₁-bis C₈-Alkylrest, vorzugsweise C₁-bis C₄-Alkylrest steht und
   - n: 1, 2 oder 3 ist,
   wobei die Reste R¹ und R² jeweils gleich oder unterschiedlich sein können;
(2) mindestens ein Polyhydrogenfluoridsalz der allgemeinen Formel (II)

   (R⁹)⁺(Hₓ₋₁Fₓ)⁻_{z} (II),

   worin
   - R⁹: für ein Metallkation aus der Reihe der Alkali-, Erdalkali oder Übergangsmetalle oder für ein Ammoniumion der Formel (R⁵)(R⁶)(R⁷)(R⁸)N⁺ steht, in der R⁵, R⁶, R⁷ und R⁸ jeweils unabhängig voneinander für H oder C₁-bis C₂₆-Alkyl-, C₃-bis C₂₆-Alkenyl- oder C₆-C₂₆-Arylreste, vorzugsweise C₁- bis C₁₂-Alkyl-, C₃- bis C₁₂-Alkenyl- oder C₆-C₁₂-Arylreste stehen, wobei R⁵, R⁶, R⁷ und R⁸ gleich oder unterschiedlich sein können, und wobei zwei dieser Reste miteinander verbunden sein können, um zusammen mit dem Stickstoffatom einen Heterocyclus zu bilden, und wobei drei der Reste und das Stickstoffatom zusammen ein Pyridiniumion bilden können,
   - x: eine ganze Zahl von 2 bis 5, vorzugsweise 2 bis 4, insbesondere 3 ist,
   - z: der Wertigkeit des kationischen Restes R⁹ entspricht,
(3) organisches Lösungsmittel, und
(4) Wasser.

Bevorzugte Alkoxysilane der Formel (I) sind solche Verbindungen, in den die Variablen die folgenden Bedeutungen haben:
- R¹: = ein Rest mit der folgenden Formel: 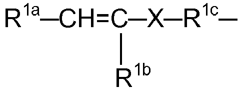 in der
R^{1a} = H oder Phenyl, vorzugsweise H ist;
R^{1b} = H oder Methyl, vorzugsweise Methyl ist;
R^{1c} = entfällt oder C₁-C₁₆-Alkyl, vorzugsweise C₁-C₃-Alkyl ist;
X = entfällt oder -CO-O- oder -CO-NH-, vorzugsweise -CO-O- oder -CO-NH- ist, wobei X entfällt, wenn R^{1c} entfällt;
- R²: = H oder C₁- bis C₂-Alkyl und
- n: = 1 oder 2,
wobei die Reste R¹ und R² jeweils gleich oder unterschiedlich sein können und wobei mehrere Rest R¹ bzw. R² vorzugsweise gleich sind.

Bevorzugte Polyhydrogenfluoride der Formel (II) sind solche Verbindungen, in den die Variablen die folgenden Bedeutungen haben:
- x: = eine ganze Zahl von 2 bis 4, vorzugsweise 3,
- z: =1,
- R⁹: = ein Ammoniumion der Formel (R⁵)(R⁶)(R⁷)(R⁸)N⁺, in der R⁵, R⁶, R⁷ und R⁸ unabhängig voneinander H, n- oder i-C₁-C₄-Alkyl bedeuten, wobei R⁵, R⁶, R⁷ und R⁸ vorzugsweise gleich sind, bevorzugt sind R⁵ = R⁶ = R⁷ = R⁸ = Butyl, insbesondere n-Butyl.

Die oben genanten bevorzugten Bedeutungen der Variablen können unabhängig voneinander gewählt werden, wobei solche Zusammensetzungen besonders bevorzugt sind, die bevorzugte Verbindungen der Formeln (I) und (II) enthalten.

Die erfindungsgemäßen Primerzusammensetzungen zeichnen sich durch ihre Fähigkeit aus, Oberflächen von Silikatkeramiken anzuätzen und gleichzeitig mit polymerisationsfähigen Gruppen zu funktionalisieren. Sie verfügen über selbstätzende Eigenschaften und vereinigen damit die Funktionen herkömmlicher Ätzmittel und Primer in einer Zusammensetzung. Das bedeutet für den Anwender eine erhebliche Arbeitserleichterung, weil das separate Ätzen und Funktionalisieren der Oberflächen entfällt und durch einen gemeinsamen Arbeitsschritt ersetzt wird. Zudem zeichnen sich die erfindungsgemäßen Primerzusammensetzungen durch eine geringe Toxizität aus, und sie sind damit auch für die intraorale Anwendung geeignet. Außerdem weisen die erfindungsgemäßen Primerzusammensetzungen eine hohe Stabilität auf. Eine Eintrübung durch Polykonden-sation der Silane findet nicht statt.

Die mit den erfindungsgemäßen Primerzusammensetzungen behandelten Oberflächen gehen einen sta-bilen, dem konventionellen Verfahren des separaten Ätzens und Primens vergleichbaren Haftverbund mit polymerisationsfähigen, radikalisch härtenden Dentalmaterialien ein, insbesondere mit dentalen Kompositen und radikalisch polymerisierbaren Zementen. Die Verbundwirkung wird durch Thermowechselbelastung nicht signifikant gemindert, was für eine dentalmedizinisch einsetzbare Haftstärke ein wesentliches Kriterium ist. Dentale Komposite sind Mischungen anorganischer Füllstoffe mit polymerisierbaren organischen Monomeren. Polymerisierbare Zemente unterscheiden sich von den Kompositen dadurch, dass sie weniger oder keine Füllstoff enthalten.

Die Primer liegen vorzugsweise in Form von homogenen Lösungen vor. Sie eignen sich besonders für dentalmedizinische Anwendungen, die einen dauerhaften Verbund polymerisierbarer Mischungen mit vorgefertigten Restaurationen auf Basis von Silikatkeramik verlangen. Die erfindungsgemäßen Zusammensetzungen ermöglichen, im Gegensatz zum Stand der Technik, das nasschemische Ätzen und das Funktionalisieren silikatischer Oberflächen in einem Arbeitsschritt. Hierzu müssen zwei Reaktionen, die üblicherweise nacheinander durchgeführt werden, nebeneinander ablaufen. Es konnte nicht erwartet werden, dass dies ohne weiteres der Fall sein wird. Vielmehr war anzunehmen, dass durch das Anätzen der Oberfläche, d.h. das chemische Abtragen von Oberflächenbereichen, bereits aufgebrachte funktionelle Gruppen wieder abgelöst werden oder aber dass oberflächlich gebundene funktionelle Gruppen den Zutritt des Ätzmittels zur Oberfläche und damit den Ätzvorgang behindern.

Das Alkoxysilan-Monomer der Formel (I), weist neben der hydrolysierbaren Alkoxygruppe -OR² mindestens einen Rest R¹ auf, der mindestens eine, bevorzugt genau eine polymerisierbare Gruppe enthält. Typischerweise handelt es sich um eine radikalisch polymerisierbare Gruppe. Bevorzugt hat das Alkoxysilan ein oder zwei R¹-Reste. Bevorzugt enthält R¹ eine ethylenisch ungesättigte Doppelbindung. Beispielsweise kann R¹ eine (Meth)acryloyl-, (Meth)acryloyloxygruppe (H₂C=C(R¹⁵)-CO-O- mit R¹⁵ = CH₃ oder H), eine (Meth)acryloylaminogruppe (H₂C=C(R¹⁶)-CO-NH- mit R¹⁶ = CH₃ oder H), eine Vinyl-, Allyl- oder Styrylgruppe enthalten, wobei die genannten Gruppen unsubstituiert oder durch geeignete Substituenten substituiert sein können. Unsubstituierte Gruppen sind bevorzugt. Bevorzugte Reste R¹ umfassen (Meth)acryloyloxyalkyl, bevorzugt (Meth)acryloyloxy-C₂-C₁₆-alkyl, besonders bevorzugt (Meth)acryloyloxypropyl; (Meth)acryloylaminoalkyl, bevorzugt (Meth)acryloyl-amino-C₂-C₁₆-alkyl, besonders bevorzugt (Meth)acryloylaminopropyl; Vinyl; Allyl und Styryl.

Geeignete Substituenten sind Aryl-, Alkylaryl-, Heteroalkyl-, Heteroaryl-, Heteroalkylaryl-, Urethan-, Halogen, Isocyanat-, Ureid-, und/oder- Imidazolinylgruppen ebenso wie Aryl-, Alkylaryl-, Heteroalkyl-, Heteroaryl-, und/oder Heteroalkylarylreste, die mit Urethan-, Halogen, Isocyanat-, Ureid-, Imidazolinylgruppen, Acryloyloxy- und / oder Methacryloyloxy-Gruppen, insbesondere mit Urethan-, Halogen, Isocyanat-, Ureid-, und/oder- Imidazolinylgruppen substituiert sind.

Der Alkylrest R² der Alkoxygruppe in Formel (I) weist 1 bis 8 C-Atome auf und ist verzweigt oder vorzugsweise geradkettig. Bevorzugt ist R² ein Methyl-, Ethyl-, n- oder i-C₃-C₈-Rest, besonders bevorzugt Methyl oder Ethyl.

Für die vorliegende Erfindung besonders geeignete Alkoxysilan-Monomere (I) sind 3-Methacryloyloxypropyltrimethoxysilan (MPTMS), 3-Methacryloyloxypropyltriethoxysilan (MPTES), Di(3-methacryloyloxypropyl)dimethoxysilan (DPDMS) und 3-Methacryloylamidopropyltrimethoxysilan (MAPTMS). Die am meisten bevorzugten Silane sind 3-Methacryloyloxypropyltrimethoxysilan und 3-Methacryloylamidopropyltrimethoxysilan.

Das Alkoxysilan-Monomer (I) ist typischerweise in der erfindungsgemäßen Primer-Zusammensetzung in einer Menge von 0,05 bis 25,0 Gew.-%, bevorzugt 0,2 bis 10,0 Gew.-% und besonders bevorzugt 0,5 bis 5,0 Gew.-% vorhanden, jeweils bezogen auf das Gesamtgewicht der Zusammensetzung.

Neben dem Alkoxysilan der Formel (I) kann der erfindungsgemäße Primer zusätzlich ein weiteres Alkoxysilan der allgemeinen Formel (la) enthalten,

(R¹³O)₃SiR¹²Si(OR¹³)₃ (Ia),

worin
- R¹²: für C₁-C₁₂-Alkylen, C₁-C₁₂-Heteroalkylen, z.B. Oligoethylenglykol, oder C₆-C₁₂-Arylen, z.B. Phenylen, steht,
- R¹³: unabhängig jeweils für H oder C₄- bis C₈-Alkyl steht,
wobei die Reste R¹³ jeweils gleich oder unterschiedlich sein können.

Bevorzugte Alkoxysilane der Formel (la) sind solche Verbindungen, in den die Variablen die folgenden Bedeutungen haben:
- R¹²: = C₄- bis C₆-Alkylen, vorzugsweise C₂-C₆-Alkylen, das ein oder mehrere, vorzugsweise ein, O-Atom(e), ein oder mehrere, vorzugsweise ein, S-Atom(e), oder vorzugsweise eine oder mehrere, vorzugsweise eine, NH-Gruppe(n) enthalten kann, oder Phenylen,

- R¹³: = H, C₁-C₈-Alkyl, vorzugsweise n- oder i-C₁-C₈-Alkyl, besonders bevorzugt Methyl oder Ethyl, wobei die Reste R¹³ unterschiedlich oder vorzugsweise gleich sein können.
Reste ohne Heteroatome sind bevorzugt.

Das Bis(alkoxysilan) der Formel (Ia), weist neben der verbrückenden Gruppe -R¹²- je Si-Atom drei hydrolysierbare Alkoxygruppen -OR¹³ auf. Die Bis(alkoxysilane) (Ia) werden stets als Mischung mit dem Alkoxysilan-Monomeren (I) verwendet. Der Alkylrest R¹³ der Alkoxygruppe in Formel (Ia) weist 1 bis 8 C-Atome auf und ist vorzugsweise ein n- oder i-C₁-C₈-Rest, besonders bevorzugt Methyl oder Ethyl.

Für die vorliegende Erfindung besonders geeignete Bis(alkoxysilane) (Ia) sind Bis(tri-ethoxysilyl)ethan, Bis(triethoxysilylethyl)benzol und N,N-Bis[3-(trimethoxysilyl)-propyl]amin. Das am meisten bevorzugte Bis(alkoxysilan) ist Bis(triethoxysilyl)ethan.

Das Bis(alkoxysilan) (Ia), ist ggf. vorzugsweise in der erfindungsgemäßen Primerformulierung in einer Menge von 0,005 bis 2,50 Gew.-%, bevorzugt 0,02 bis 1,0 Gew.-% und besonders bevorzugt 0,05 bis 0,5 Gew.-% vorhanden, jeweils bezogen auf das Gesamtgewicht der Zusammensetzung.

Das polyhydrogenfluoridhaltige Salz der allgemeinen Formel (II) zeichnet sich durch eine gute Löslichkeit im eingesetzten Lösemittelgemisch aus. Bevorzugt sind solche Verbindungen, die in den unten genannten Konzentrationen im verwendeten Lösungsmittel löslich sind. R⁵, R⁶, R⁷ und R⁸ sind Teil eines Ammonium-Ions und vorzugsweise gleich oder unabhängig voneinander H, n- oder i-Alkan oder -Alkenyl mit einer Kettenlänge von C₁ bis C₂₆, vorzugsweise C₃₋₁₂. R⁵, R⁶, R⁷ und R⁸ können auch als Teil einer cyclischen Ammoniumverbindung miteinander verbunden sein. Bevorzugte Reste R⁵, R⁶, R⁷ und R⁸ sind H, C₁ bis C₄ n- oder i-Alkane, wobei R⁵, R⁶, R⁷ und R⁸ gleich sind. Besonders bevorzugt ist R⁵ = R⁶ = R⁷ = R⁸ = Butyl, insbesondere n-Butyl. Das in Formel (II) enthaltene Polyhydrogenfluorid-Anion ist gekennzeichnet durch x = 2 - 5, vorzugsweise 2 - 4 wobei x = 3 besonders bevorzugt ist.

Die Gruppen R⁵ bis R⁸ können substituiert oder vorzugsweise unsubstituiert sein. Bevorzugte Substituenten sind Halogen, insbesondere Chlor und Brom, sowie aromatische und heteroaromatische Gruppen, vorzugsweise Phenyl und Pyridinyl. Die Gruppen R⁵ bis R⁸ sind ggf. vorzugsweise durch 1 bis 12, besonders bevorzugt 1 bis 6 und insbesondere 1 bis 4 Halogenatome oder 1 bis 4 aromatische oder heteroaromatische Einheiten substituiert.

In einer weiteren Ausführungsform ist (R⁹) ein Metallkation. Hierbei handelt es sich um biologisch kompatible Metallkationen, vorzugsweise um Natrium oder Kalium.

Das Polyhydrogenfluoridhaltige Salz (II) ist typischerweise in der erfindungsgemäßen Primerzusammensetzung in einer Menge von 1 bis 25,0 Gew.-%, bevorzugt 2,0 bis 15,0 Gew.-% und besonders bevorzugt 5,0 bis 10,0 Gew.-% vorhanden, jeweils bezogen auf das Gesamtgewicht der Zusammensetzung.

Komponente (3) der erfindungsgemäßen Primerzusammensetzung ist ein organisches Lö-sungsmittel oder ein Lösemittelgemisch, bestehend aus physiologisch verträglichen Lösungsmitteln. Geeignete Lösungsmittel sind beispielsweise Alkohole, Ketone und Ester, wobei Methanol, Ethanol, n-Propanol, i-Propanol, t-Butanol, Ethylacetat, Aceton, Methylethylketon und deren Mischungen bevorzugt sind. Besonders bevorzugt ist Ethanol.

Typischerweise enthält die Primerzusammensetzung 25 bis 98,5 Gew.-%, bevorzugt 35 bis 75 Gew.-% und besonders bevorzugt 45 bis 55 Gew.-% organisches Lösungsmittel, jeweils bezogen auf das Gesamtgewicht der Zusammensetzung.

Komponente (4) ist das zur Verbesserung der Ätzwirkung des Polyhydrogenfluoridsalzes nötige Wasser. Typischerweise enthält die Primerformulierung 25 bis 98,5 Gew.-%, bevorzugt 35 bis 75 Gew.-% und besonders bevorzugt 40 bis 75 Gew.-% Wasser, jeweils bezogen auf das Gesamtgewicht der Zusammensetzung.

Die erfindungsgemäßen Primerzusammensetzungen eignen sich besonders zur Behandlung der Oberflächen von dentalen Restaurationen mit silikatkeramischen Oberflächen. Das simultane Ätzen und Primen von Silikatkeramiken mit der erfindungsgemäßen selbstätzenden Primerzusammensetzungen erfordert nur vier Arbeitsschritte, (1) das Auftragen des Primers auf die Oberfläche, (2) das Einwirken lassen des Primers, (3) das Abspülen des selbstätzenden Primers mit Wasser oder einem geeigneten Lösungsmittel und (4) das Trocknen der Oberfläche. Der Einsatz eines selbstätzenden Primers reduziert demnach die Zahl der für die Vorbereitung silikatischer Keramiken nötiger Arbeitsschritten deutlich und ermöglicht so ein schnelleres und weniger fehleranfälliges Vorgehen. Die erfindungsgemäße Primerzusammensetzung enthält vorzugsweise kein freies HF, d.h. sie kommt ohne Beimischung oder *in situ* Bildung freier Flusssäure aus. Das erhöht bei versehentlichem Kontakt die Arbeitssicherheit hinsichtlich auftretender toxischer oder gewebezerstörender Wirkungen.

Neben dem Silan der Formel (I) und dem Polyhydrogenfluoridsalz (II) kann die erfindungsgemäße Primerzusammensetzung als Komponente (5) zusätzlich ein Phosphorsäure- oder Pyrophosphorsäureester-Monomer der Formel (III) enthalten:

O=P(OR³)ₘ(OR⁴)₃₋ₘ (III),

worin
- R³: für einen organischen Rest steht, der mindestens eine ethylenisch ungesättigte, polymerisierbare Gruppe aufweist,
- R⁴: für H, SiR₃, P(=O)(OR¹⁴)₂ oder C₁- bis C₁₆-Alkyl, vorzugsweise H steht, wobei R¹⁴ für H, SiR'₃ oder C₄- bis C₁₆-Alkyl, vorzugsweise H steht und wobei R und R' unabhängig voneinander jeweils C₁ bis C₄-Alkyl und insbesondere Methyl sind, und
- m: 1 oder 2 ist,
wobei mehrere Reste R³ und R⁴ jeweils gleich oder unterschiedlich sein können.

Bevorzugte Verbindungen der Formel (III) sind solche Verbindungen, in den die Variablen die folgenden Bedeutungen haben:
- R³: = ein Rest mit der folgenden Formel: 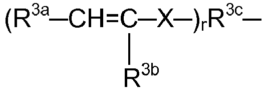 in der
R^{3a} = H oder Phenyl, vorzugsweise H ist;
R^{3b} = H oder Methyl, vorzugsweise Methyl ist;
R^{3c} = entfällt oder C₁-C₁₆-Alkyl, vorzugsweise C₂-C₃-Alkyl ist;
X = entfällt oder -CO-O- oder -CO-NH-, vorzugsweise -CO-O- oder -CO-NH- ist, wobei X entfällt, wenn R^{3c} entfällt;
r = 1 oder 2;
- R⁴: = Methyl, Ethyl, vorzugsweise H;
- m: = 1,
wobei die Reste R⁴ unterschiedlich oder vorzugsweise gleich sind.

Durch die Zugabe der Verbindung (III) wird die Haftung des Primers an Oxidkeramiken und Nichtedelmetallen verbessert. Auf diese Weise wird das Anwendungsspektrum der erfingsgemäßen Primerzusammensetzung auf solche Materialien erweitert. Dies ermöglicht die Verwendung der Zusammensetzung zur Verbesserung der Verbundwirkung zwischen einem radikalisch härtenden Dentalmaterial und einer Vielzahl von anderen dentalen Restaurationsmaterialien. Hierdurch kann bei der klinischer Anwendung im Vergleich zu substratspezifischen Primern die Verwechslungsgefahr und das Risiko des klinischen Misserfolgs reduziert werden. Ein besonderer Vorteil solcher universell einsetzbarer Primer ist, dass sie bei Restaurationen eingesetzt werden können, die unterschiedliche Materialien enthalten. Die ist zum Beispiel regelmäßig bei der Reparatur frakturierten Keramikverblendungen der Fall, wo mehrere Substrate auf engem Raum in direkter Nachbarschaft nebeneinander vorliegen.

Das Phosphorsäureester-Monomer der allgemeinen Formel (III) weist mindestens einen Rest R³ auf, der mindestens eine, bevorzugt eine oder zwei polymerisierbare Gruppen enthält. Typischerweise handelt es sich um (eine) radikalisch polymerisierbare Gruppe(n). Vorzugsweise hat der Phosphorsäureester genau einen R³-Rest. Pyrophosphate (R⁴= P(=O)(OR¹⁴)₂) werden in Anwesenheit von Wasser im Laufe der Zeit zu Phosphaten der Formel (III) hydrolysiert.

Bevorzugt enthält R³ in Formel (III) mindestens eine ethylenisch ungesättigte Doppelbindung. Beispielsweise kann R³ mindestens eine (Meth)acryloyloxygruppe, eine (Meth)acryloylaminogruppe, eine Vinyl-, Allyl- oder Styrylgruppe oder eine Kombination derselben enthalten. Bevorzugte Reste R³ umfassen (Meth)acryloyloxyalkyl, bevorzugt (Meth)acryloyloxy-C₂-C₁₆-alkyl, besonders bevorzugt (Meth)acryloyloxy-C₄-C₁₄-alkyl, ganz besonders bevorzugt (Meth)acryloyloxy-C₆-C₁₀-alkyl; Di(meth)acryloyloxyalkyl, bevorzugt Di(meth)acryloyloxy-C₂-C₁₆-alkyl, besonders bevorzugt Di(meth)acryloyloxy-C₂-C₁₀-alkyl, ganz besonders bevorzugt Di(meth)acryl-oyloxyisopropyl; (Meth)acryloylaminoalkyl, bevorzugt (Meth)acryloylamino-C₂-C₁₆-alkyl, besonders bevorzugt (Meth)acryloylamino-C₄-C₁₄-alkyl, ganz besonders bevorzugt (Meth)acryloylamino-C₆-C₁₀-alkyl.

Der R⁴-Rest ist vorzugsweise ausgewählt aus H, Silyl, bevorzugt SiMe₃, und C₁-bis C₁₆-Alkyl, wobei der Alkylrest verzweigt oder vorzugsweise geradkettig sein kann, bevorzugt ist R⁴ H, Methyl, Ethyl oder ein n- oder i-C₃-C₁₆-Rest. In einer besonders bevorzugten Ausführungsform ist R⁴ gleich H, wobei die Dihydrogenphosphate (Phosphorsäuremonoester) die am meisten bevorzugten Phosphorsäureester-Monomere darstellen.

Für die vorliegende Erfindung besonders geeignete Phosphorsäureester-Monomere (III) sind 1-Methacryloyloxydecan-10-phosphat (MDP), 1-Methacryloyloxyhexan-6-phosphat (MHP), 1-Methacryloylamidodecan-10-phosphat (MADP), 1-Acryloyl-amidohexan-6-phosphat (AAHP), 13-Dimethacryloyloxypropan-2-phosphat (DMPP) und 1,3-Dimethacryloylamidopropan-2-phosphat (DMAPP). Das am meisten bevorzugte Phosphorsäureester-Monomer ist das 1-Methacryloyloxydecan-10-phosphat.

Das Phosphorsäureester-Monomer (III), wird ggf. vorzugsweise in einer Menge von 0,05 bis 25,0 Gew.-%, besonders bevorzugt 0,2 bis 10,0 Gew.-% und ganz besonders bevorzugt 0,5 bis 5,0 Gew.-% verwendet, jeweils bezogen auf das Gesamtgewicht der Zusammensetzung.

Anstelle oder vorzugsweise zusätzlich zu dem Phosphorsäure- oder Pyrophosphorsäureester-Monomer der Formel (III) kann die erfingsgemäße Primerzusammensetzung ein oder mehrere Phosphonsäure- oder Polyphosphonsäure-Monomere der allgemeinen Formel (IIIa) enthalten:

R¹¹[P(=O)(OR¹⁰)₂]ₙ (IIIa)

worin
- R¹¹: für einen organischen Rest steht, der mindestens eine ethylenisch ungesättigte, polymerisierbare Gruppe aufweist,
- R¹⁰: für einen Rest ausgewählt aus H, SiR"₃ oder C₁-bis C₁₆-Alkyl steht, wobei R" C₁ bis C₄-Alkyl, vorzugsweise Methyl ist,
- n: 1, 2, 3 oder 4, vorzugsweise 1 oder 2 und insbesondere 1 ist.
wobei die Reste R¹⁰ jeweils gleich oder unterschiedlich sein können.

Bevorzugte Verbindungen der Formel (IIIa) sind solche Verbindungen, in den die Variablen die folgenden Bedeutungen haben:
- R¹¹: = ein Rest der allgemeinen Formel. 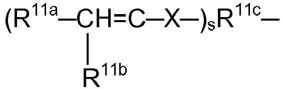 in der
R^{11a} = H oder Phenyl, vorzugsweise H ist;
R^{11b} = H oder Methyl, vorzugsweise Methyl ist;
R^{11c} = entfällt oder C₁-C₁₆-Alkyl, vorzugsweise C₂-C₃-Alkyl ist;
X = entfällt oder -CO-O- oder -CO-NH-, vorzugsweise -CO-O- oder-CO-NH- ist, wobei X entfällt, wenn R^{11c} entfällt;
s = 1 oder 2 ist;
- R¹⁰: = Methyl, Ethyl, vorzugsweise H;
- m': = 1 oder 2, vorzugsweise 1,
- p: = 1,
wobei die Reste R¹⁰ unterschiedlich oder vorzugsweise gleich sind.

Das Phosphonsäure-Monomer der allgemeinen Formel (IIIa) weist mindestens einen Rest R¹¹ auf, der mindestens eine, bevorzugt eine oder zwei polymerisierbare Gruppen enthält. Typischerweise handelt es sich um (eine) radikalisch polymerisierbare Gruppe(n). Vorzugsweise hat die Phosphonsäure (IIIa) genau einen R¹¹-Rest.

Bevorzugt enthält R¹¹ in Formel (IIIa) mindestens eine ethylenisch ungesättigte Doppelbindung. Beispielsweise kann R³ mindestens eine (Meth)acryloyloxygruppe, eine (Meth)acryloylaminogruppe, eine Vinyl-, Allyl- oder Styrylgruppe oder eine Kombination derselben enthalten. Bevorzugte Reste R³ umfassen (Meth)acryloyloxyalkyl, bevorzugt (Meth)acryloyloxy-C₂-C₁₆-alkyl, besonders bevorzugt (Meth)acryloyloxy-C₄-C₁₄-alkyl, ganz besonders bevorzugt (Meth)acryloyloxy-C₆-C₁₀-alkyl; Di(meth)acryloyloxyalkyl, bevorzugt Di(meth)acryloyloxy-C₂-C₁₆-alkyl, besonders bevorzugt Di(meth)acryloyloxy-C₂-C₁₀-alkyl, besonders bevorzugt Di(meth)acryl-oyloxyisopropyl; (Meth)acryloylaminoalkyl, bevorzugt (Meth)acryloylamino-C₂-C₁₆-alkyl, besonders bevorzugt (Meth)acryloylamino-C₄-C₁₄-alkyl, ganz besonders bevorzugt (Meth)acryloylamino-C₆-C₁₀-alkyl.

Der R¹⁰-Rest ist vorzugsweise ausgewählt aus H, Silyl, bevorzugt SiMe₃, und C₁- bis C₁₆-Alkyl, wobei der Alkylrest verzweigt oder vorzugsweise geradkettig sein kann, bevorzugt ist R¹⁰ H, Methyl, Ethyl oder ein n- oder i-C₃-C₁₆-Rest. In einer besonders bevorzugten Ausführungsform ist R¹⁰ gleich H, wobei die Dihydrogenphosphonate (Phosphondisäure) die am meisten bevorzugten Phosphonsäure-Monomere sind.

Für die vorliegende Erfindung besonders geeignete Phosphonsäure-Monomere (IIIa) sind 1-Methacryloyloxydecan-10-phosphonat, 9-Methacryloyloxynonylphosphonsäure, 2-[4-(Dihydroxyphosphoryl)-2-oxabutyl]-acrylsäureethylester-phosphonat. Das am meisten bevorzugte Phosphonsäure-Monomer ist 9-Methacryloyloxynonylphosphonsäure.

Das Phosphonsäure-Monomer (IIIa) wird ggf. vorzugsweise in einer Menge von 0,05 bis 25,0 Gew.-%, besonders bevorzugt 0,2 bis 10,0 Gew.-% und ganz besonders bevorzugt 0,5 bis 5,0 Gew.-% verwendet, jeweils bezogen auf das Gesamtgewicht der Zusammensetzung.

Die oben gezeigten Formeln umfassen nur solche Verbindungen, die mit der chemischen Äquivalenzlehre vereinbar sind. Erfindungsgemäß sind dabei solche Verbindungen bevorzugt, in denen alle Variablen eine der bevorzugten und insbesondere eine der besonders bevorzugten Bedeutungen haben. Ebenso sind solche Zusammensetzungen bevorzugt, die eine Kombination von bevorzugten bzw. besonders bevorzugten Komponenten enthalten, wobei die erfindungsgemäße Primerzusammensetzung jeweils eine der genannten Komponenten oder eine Mischung aus mehreren Substanzen des jeweiligen Typs enthalten kann.

Weiterhin kann die erfindungsgemäße Primerzusammensetzung zusätzliche Hilfsmittel (6) enthalten, etwa Netzmittel, Detergenzien, wie z.B. nichtionische, anionische und/oder kationische Tenside, bevorzugt nichtionische und kationische Tenside, Entschäumer, Stabilisatoren und weitere Hilfsmittel, wie etwa antimikrobielle Zusätze, Duftstoffe, Farbstoffe und Konservierungsmittel. Die erfindungsgemäße Primerzusammensetzung kann polymere Verdickungsmittel, z.B. geeignet lösliche Polyvinylverbindungen, Polymethacrylate, Polyacrylate, Polyether, Polyamine, Polysilicate, und Polysaccharide, sowie Thixotropieagenzien und Rheologiemodifizierer enthalten.

Besonders bevorzugt sind Zusammensetzungen, die als Hilfsmittel Verdicker, Farbstoff(e), Radikalstabilisator(en) und / oder Tensid enthalten.

Bevorzugte Hilfsstoffe sind nichtionische Verdickungsmittel und Farbstoffe in Anteilen von jeweils 0,001 - 5 Gew.-%, wobei der Gesamtanteil an Hilfsmittels (6) in der Formulierung im Bereich von 0,001 bis 10 Gew.-% liegt.

Gemäß einer bevorzugten Ausführungsform enthält die Primerzusammensetzung der vorliegenden Erfindung:
(1) 0,05 bis 25,0 Gew.-%, bevorzugt 0,2 bis 10,0 Gew.-% und besonders bevorzugt 0,5 bis 5,0 Gew.-% Alkoxysilan-Monomer (I) und ggf. 0,005 bis 2,5 Gew.-%, bevorzugt 0,02 bis 1,0 Gew.-% und besonders bevorzugt 0,05 bis 0,5 Gew.-% des Alkoxysilans (la);
(2) 1,0 bis 25,0 Gew.-%, bevorzugt 2,0 bis 15,0 Gew.-% und besonders bevorzugt 5,0 bis 10,0 Gew.-% Polyhydrogenfluoridsalz (II);
(3) 25 bis 98,5 Gew.-%, bevorzugt 35 bis 75 Gew.-% und besonders bevorzugt 45 bis 55 Gew.-% organisches Lösungsmittel;
(4) 25 bis 98,5 Gew.-%, bevorzugt 35 bis 75 Gew.-% und besonders bevorzugt 40 bis 75 Gew.-% Wasser;
(5) ggf. 0,05 bis 25,0 Gew.-%, bevorzugt 0,2 bis 10,0 Gew.-% und besonders bevorzugt 0,5 bis 5,0 Gew.-% Phosphorsäureester-Monomer (III) und ggf. 0,05 bis 25,0 Gew.-%, bevorzugt 0,2 bis 10,0 Gew.-% und besonders bevorzugt 0,5 bis 5,0 Gew.-% Phosphonsäure-Monomer (IIIa);
(6) ggf. 0,001 bis 10%, bevorzugt 0,1 bis 7,5%, besonders bevorzugt 1,0 bis 5% Hilfsmittel, jeweils bezogen auf das Gesamtgewicht der Zusammensetzung.

Ganz besonders bevorzugt sind erfindungsgemäß die Primerzusammensetzungen A, B und C, die die folgenden Bestandteile enthalten:
(A) eine Kombination von (I) 3-Methacryloxypropyltrimethoxysilan, (II) Tetrabutylammoniumdihydrogentrifluorid und (III) 1-Methacryloyloxydecan-10-phosphat;
(B) eine Kombination von (I) Methacrylsäure-(3-trimethoxysilylpropyl)amid; (II) Tetrabutylammonium-Hydrogendifluorid und (III) 1-Methacryloylamidodecan-10-phosphat.
(C) eine Kombination von (I) 3-Methacryloxypropyltrimethoxysilan., (II) Ammoniumhydrogendifluorid und (III) 1-Methacryloyloxydecan-10-phosphat.

Vorzugsweise werden diese Komponenten in den zuvor genannten Mengen und wahlweise mit den genannten optionalen Additiven eingesetzt.

Die Erfindung betrifft auch die Verwendung der Haftvermittlerzusammensetzung in der Zahnheilkunde und Zahntechnik, insbesondere die Verwendung zur adhäsiven Verbindung von metallischen oder keramischen Dentalmaterialien, besonders silikatkeramischen Dentalwerkstoffen mit radikalisch härtenden Dentalmaterialien, bevorzugt Zementen, Kompositen und auf Kompositen basierenden Befestigungsmaterialien (Kompositzemente), aber auch füllstofffreien Methacrylsäurederivaten und Mischungen von Methacrylsäurederivaten.

Die erfindungsgemäßen Zusammensetzungen eignen sich besonders zur Verwendung als selbstätzende Primer für silikatische Keramiken, wie etwa Feldspat, Quarz, Leucit- oder Lithiumdislikat-basierende Keramiken bzw. sonstige glashaltige Keramiken. Zusammensetzungen, die ein Phosphorsäureester- (III) und/oder Phosphonsäure-Monomer (IIIa) enthalten, eignen sich auch zum Primen nichtsilikatischer Keramiken, wie etwa Yttrium-stabilisiertem Zirkonoxid, Aluminiumoxid, und Nichtedelmetallen, wie etwa Titan, titanhaltige Legierungen, Chrom-haltige, Nickel-haltige oder Kobalt-haltige Dentallegierungen. Dentale Restaurationen aus den genannten Werkstoffen können nach der oberflächlichen Behandlung mit der erfindungsgemäßen Primerformulierung unter Verwendung radikalisch härtende Befestigungsmaterialien sicher am natürlichen Zahn befestigt werden. Es wird ein guter Verbund - auch nach Thermowechselbelastung - erzielt, was auf eine hohe Haltbarkeit des Verbundes unter oralen Bedingungen hindeutet.

Die Erfindung betrifft auch die Verwendung der erfindungsgemäßen Primerzusammensetzungen in der Zahnheilkunde und Zahntechnik, insbesondere die Verwendung als Haftvermittler zur adhäsiven Verbindung keramischer Dentalwerkstoffen mit radikalisch härtenden Dentalmaterialien, bevorzugt Zementen, Kompositen und auf Kompositen basierenden Befestigungsmaterialien. Die erfindungsgemäßen Zusammensetzungen eignen sich zur intraoralen Anwendung durch den Zahnarzt (therapeutische Anwendung) und die extraorale Anwendung (nicht-therapeutische Anwendung).

Zur Behandlung mit den erfindungsgemäßen Primerzusammensetzungen werden silikatische Keramiken nach der Anfertigung gereinigt. Beispielsweise können Herstellrückständen (z.B. Gussform- oder Schleifmittelrückstände) durch die dentaltechnisch üblichen Verfahren (Dampfstrahlen, Ultraschallbad oder Wasserstrahl) entfernt werden. Anschließend werden die Keramiken getrocknet, beispielsweise mit einem Luftbläser von oberflächlich anhaftendem Wasser befreit. Im Anschluss wird die Primerformulierung mit einer geeigneten Applikationsvorrichtung (z.B. Pinsel, Flockkanüle) auf die gesamte Klebefläche aufgetragen und ohne weitere Agitation für vorzugsweise 15 bis 300 sec auf der Keramik belassen. Im Anschluss wird überschüssiger Primer mit einem Wasserstrahl abgespült und die Keramik getrocknet, z.B. mit einem Luftbläser trocken geblasen. Das weitere Zementierungsverfahren (Auftragen von Zement, Einpassen der Keramik in die Kavität / auf den Zahnstumpf, Zementvorhärtung, Überschussentfernung, endgültige Zementhärtung) erfolgt nach der für die Restauration passenden und dentalmedizinisch üblichen Vorgehensweise.

Zur Beschichtung oxidkeramischer Keramiken und nichtedelmetallischer Restaurationen mit den erfindungsgemäßen Primerzusammensetzungen werden oxidkeramische und nichtedelmetallische Restaurationen nach der Anfertigung gereinigt. Beispielsweise können Herstellrückständen (z.B. Gussform- oder Schleifmittelrückstände) durch die dentaltechnisch üblichen Verfahren (Dampfstrahlen, Ultraschallbad oder Wasserstrahl) entfernt werden. Anschließend werden die Keramiken getrocknet, beispielsweise mit einem Luftbläser von oberflächlich anhaftendem Wasser befreit. Im Anschluss wird die Restauration nach dem vom Hersteller des Restaurationsmaterials vorgegeben Sandstrahlverfahren oberflächlich angeraut. Üblich sind hierbei die Angabe der Strahlmittelkörnung und des Strahldrucks. Gefolgt wird der Strahlvorgang üblicherweise einem weiteren Reinigungsschritt zur Entfernung der Strahlmittelrückstände, beispielsweise in einem Ultraschallbad. Danach wird die Restauration getrocknet, beispielsweise durch einen Luftstrom von oberflächlich anhaftendem Wasser befreit. Dann wird Primerzusammensetzung mit einer geeigneten Applikationsvorrichtung (z.B. Pinsel, Flockkanüle) auf die gesamte Klebefläche aufgetragen und ohne weitere Agitation vorzugsweise für 30 bis 300 sec auf der Keramik oder der Metalloberfläche belassen. Im Anschluss wird überschüssiger Primer mit einem Wasserstrahl abgespült und die Restauration getrocknet, z.B. mit einem Luftbläser trocken geblasen. Das weitere Zementierungsverfahren (Auftragen Zement, Einpassen der Keramik in die Kavität / auf den Zahnstumpf, Zementvorhärtung, Überschussentfernung, endgültige Zementhärtung) erfolgt nach der für die Restauration passenden und dentalmedizinisch üblichen Vorgehensweise.

Die mit der erfingsgemäßen Primerzusammensetzung behandelten Silikatkeramiken, Oxidkeramiken und Metallrestaurationen sind ebenfalls Gegenstand der Erfindung. Die Erfindung wird im Folgenden anhand von Beispielen näher erläutert.

### Ausführungsbeispiele

### Beispiel 1

### Herstellung von Haftvermittlern (Primern)

Die in der folgenden Tabelle 1 angegebenen Primer A bis G wurden durch Zusammengeben der Komponenten und Rühren derselben bis zur Homogenität hergestellt.

**Tabelle 1: Zusammensetzung der Primer**

| **Bestandteil** | **Primer [Gew.-%]** | | | | | | |
|---|---|---|---|---|---|---|---|
| | **A** | **B** | **C** | **D** | **E** | **F** | **G** |
| **Komponente (1)** | | | | | | | |
| 3-Methacryloxypropyltrimethoxysilan¹ | 1,0 | 1,0 | 1,0 | 10,0 | | 3,0 | 3,0 |
| Methacrylsäure-(3-trimethoxysilylpro-pyl)amid² | | | | | 1,0 | | |
| Bis(triethoxysilyl)ethan ⁵ | | | | | | | 0,3 |

| **Komponente (5)** | | | | | | | |
|---|---|---|---|---|---|---|---|
| 1-Methacryloyloxydecan-10-phosphat³ | | 1,0 | 1,0 | | | 3,0 | 3,0 |
| 1-Methacryloylamidodecan-10-phosphat⁴ | | | | | 1,0 | | |

| **Komponente (2)** | | | | | | | |
|---|---|---|---|---|---|---|---|
| Tetra-n-butylammoniumhydrogendi-fluorid⁵ | 10 | | | | | | |
| Ammoniumhydrogendifluorid⁶ | | 10 | | | | | |
| Tetra-n-butylammoniumdihydrogentri-fluorid⁷ | | | 10 | 20 | 10 | 10 | 10 |
| **(3) Ethanol** | 45 | 44 | 44 | 35 | 44 | 41 | 40,7 |
| **(4) Wasser** | 44 | 44 | 44 | 35 | 44 | 42 | 42 |

| **(6) Hilfsmittel** | | | | | | | |
|---|---|---|---|---|---|---|---|
| Verdicker (Methocel 90HG)⁸ | | | | | | 1,0 | 1,0 |

| **Bestandteil** | **Primer [Gew.-%]** | | | | | | |
|---|---|---|---|---|---|---|---|
| | **A** | **B** | **C** | **D** | **E** | **F** | **G** |
| Farbstoff (Fast Green FCF)⁶ | | | | | | | 0,06 |
| Farbstoff (Coomassie Violet)⁶ | | | | | | 0.06 | |

| | | | | | | | |
|---|---|---|---|---|---|---|---|
| ¹ kommerziell erhältlich von Fa. Sigma Aldrich ² wurde gemäß Beispiel 1 der EP 2 229 930 A1 hergestellt ³ wurde gemäß US 4,612,384 in einer Ausbeute von 75% und einer Reinheit von 95% (bestimmt durch HPLC) hergestellt ⁴ wurde gemäß EP 1 674 066 in einer Ausbeute von 69% und einer Reinheit von 93% (bestimmt durch HPLC) hergestellt ⁵ kommerziell erhältlich von Fa. ABCR, Karlsruhe, Deutschland ⁶ kommerziell erhältlich von Fa. Sigma-Aldrich, Buchs, Schweiz ⁷ kommerziell erhältlich von Fa. TCI Europe, Zwijndrecht, Belgien ⁸ Polysaccharid; kommerziell erhältlich von Fa. Sigma-Aldrich, Buchs, Schweiz | | | | | | | |

### Beispiel 2

### Bestimmung der Haftwerte auf unterschiedlichen dentalen Restaurationsmaterialien

Zur Bestimmung der Haftwerte wurde eine Abzugsanordnung angewendet wie sie in der Literatur beschrieben ist (M. Kern, V.P. Thompson, J. Prost. Dent. 1995 73(3): 240-249; M. Kern, V.P. Thompson, "Eine einfache Versuchsanordnung zur universellen Prüfung des Klebeverbundes im axialen Zugtest", Dtsch Zahnärztl Z 1993, 48: 769-772).

### Materialien

Die erfindungsgemäßen Primerzusammensetzungen A - F wurden mit den folgenden kommerziell erhältlichen Keramik- bzw. Metallprimern verglichen:
- MBP:: Monobond Plus (Ivoclar Vivadent AG, Schaan, Liechtenstein) auf Basis von 3-Methacryloxypropyltrimethoxysilan, 10-Methacryloyloxydecyldihydrogenphosphat und Liponsäure-2-ethoxycarbonylallylester
- MBS:: Monobond-S Primer von Ivoclar Vivadent AG, Schaan, Liechtenstein (auf Basis von 3-Methacryloxypropyltrimethoxysilan)
- CP:: Clearfil Ceramic Primer (Fa. Kuraray Europe GmbH, Deutschland) auf Basis von 3-Methacryloxypropyltrimethoxysilan und 10-Methacryloyloxydecyl-dihydrogenphosphat
- ZP:: Z-Prime (Fa. BISCO, USA) auf Basis 10-Methacryloyloxydecyl-dihydrogenphosphat

Die erfindungsgemäßen Primerzusammensetzungen zur Verwendung als Haftvermittler wurden auf folgenden Restaurationsmateria-lien getestet:
- E.max:: Lithiumdisilikat-Keramik E.max CAD (Fa. Ivoclar Vivadent AG, LI),
- EMpress:: Leucit-Keramik Empress (Fa. Ivoclar Vivadent AG, Liechtenstein),
- ZirCAD:: Zirkonoxidkeramik ZirCAD (Fa. Ivoclar Vivadent AG, Liechtenstein)
- Al-Cube:: Aluminiumoxid-Keramik Al-Cube (Fa. Vita, Deutschland)
- Titan:: Reintitan (Tritan, Fa. Dentaurum, Deutschland).

### Vorbereitung der Probekörperoberfläche

Die würfelförmigen Prüfkörper wurden unter Wasserkühlung mit SiC-Schleifpapier der Korngrößen P120 und P400 plan geschliffen und anschließend mit P1000 poliert.

### Ätzen von Silikatkeramik mit HF für Vergleichsmessungen

Für Referenzmessungen mit HF-Vorbehandlung wurde eine polierte und ultraschallgereinigte Lithiumdisilikat-Glaskeramik (E.max CAD, Fa. Ivoclar Vivadent, Liechtenstein) agitationsfrei für 20 s mit Flusssäure-Gel (Ceramic Etch, Fa. Ivoclar Vivadent AG, Liechtenstein) kontaktiert, mit destilliertem Wasser abgespült und die Oberfläche mit ölfreier Druckluft trockengeblasen. Parallel wurde eine leucitverstärkte Silikatkeramik (Empress, Fa. Ivoclar Vivadent, Liechtenstein) agitationsfrei für 60 s mit dem Flusssäure-Gel (Ceramic Etch, Fa. Ivoclar Vivadent AG, Liechtenstein) kontaktiert, mit destilliertem Wasser abgespült und die Oberfläche mit ölfreier Druckluft trockengeblasen. Die Proben wurden dann staubgeschützt bis zum Einsatz aufbewahrt.

### Sandstrahlen der oxidkeramischen und metallischen Proben

Die oxidkeramische Oberflächen Zirkonoxid (ZirCAD, Fa. Ivoclar Vivadent, Liechtenstein) und Aluminiumoxid (Al-Cube, Fa. Vita, Deutschland) sowie Reintitan (Tritan, Fa. Dentaurum, Deutschland) wurden mit 50 µm Aluoxid-Strahlmittel (Korox 50) bei 2,5 x 10⁵ Pa (2,5 bar) Druck aus ca.1-2 cm Abstand für 15 s angeraut. Die Proben wurden anschließend auf dem Rand stehend (d.h. Haftfläche senkrecht ohne Wand-oder Bodenkontakt) für 10 min in i-Propanol in einem Ultraschallbad gereinigt. Nach Entnahme aus dem i-Propanol wurden die Proben mit Druckluft trockengeblasen und staubgeschützt bis zum Einsatz aufbewahrt.

### Applikation der nicht erfindungsgemäßen Primerformulierungen auf geätzte oder sandgestrahlte Oberflächen

Für Referenzmessungen mit kommerziell erhältlichen Haftvermittlern wurden die geätzten oder sandgestrahlten Probekörper gemäß der jeweiligen Gebrauchsinformation mit dem Produkt behandelt.

### Applikation der erfingsgemäßen Primerzusammensetzungen auf polierte oder sandgestrahlte Oberflächen

Zum Test der erfindungsgemäßen Primerzusammensetzungen wurden durch den oben beschriebenen Vorbereitungsprozess plan geschliffene und polierte Silikatkeramiken, oder sandgestrahlte Oxikeramiken und Metalle mit einer der erfindungsgemäßen Primerzusammensetzungen A-G kontaktiert. Dazu wurden die Proben mit einem mit der Primerzusammensetzung getränkten Microbrushpinsel einmalig satt bestrichen und die Flüssigkeit 60 s einwirken gelassen. Anschließend wurde überstehende Flüssigkeit mit Leitungswasser abgespült und danach die Oberfläche mit ölfreier Druckluft trockengeblasen.

### Messung der Zughaftun

Auf die geprimten Oberflächen wurde, wie in Dtsch Zahnärztl Z (1993) 48, 769-772 beschrieben, eine mit lichtpolymerisiertem Komposit (Multicore Flow, Ivoclar Vivadent AG, Schaan, Liechtenstein) gefüllte Plexiglashülse aufgebracht. Dazu wurde die Hülse am zu haftenden Ende mit einem Tropfen Dentalzement (Multilink Automix, Ivoclar Vivadent AG, Schaan, Liechtenstein) belegt und mittels einer Anpressapparatur auf den Keramikprobekörper gedrückt. Dann wurde der Zement durch 2 x 20 s Bestrahlung mit einer Polymerisationslampe (Typ BluePhase G2, Fa. Ivoclar Vivadent, Liechtenstein) ausgehärtet und die Proben für 24 h bei 37 °C in Wasser gelagert. Danach wurde die Zughaftung mit der in der genannten Literatur beschriebenen Probenhalterung in einer Universal-Test-Maschine (Typ Z010, Zwick-Roell, Ulm, Deutschland) bestimmt.

Zur Simulierung einer Dauerbelastung wurden die Prüfkörper auch einer Thermowechselbelastung unterzogen. Hierzu wurden die Prüfkörper vor Messung der Zughaftung 10'000 mal von 5°C kaltem Wasser in 55 °C warmes Wasser und zurück übertragen und jeweils für 60 s in dem Wasser belassen.

### Ergebnisse

Die Ergebnisse sind in Tabelle 2 zusammengefasst. Wie die Haftwerte in der Tabelle 2 zeigen, zeichnen sich die erfindungsgemäßen Primerzusammensetzungen A, B, C, D, E, F und G durch einen sehr guten Verbund zu den jeweils getesteten Substratoberflächen aus und verfügen (wo getestet) über eine gute, den kommerziell erhältlichen Produkten vergleichbare Thermolastbeständigkeit. Auf Silikatkeramik wurden ohne vorherige HF-Ätzung Haftwerte erzielt, die mit denen kommerziellen Formulierungen nach HF-Ätzung vergleichbar oder sogar besser sind. Neben diesem Vorteil wurde mit den phosphathaltigen Formulierungen C, E und F ein mit den kommerziellen Primern MBP und CP vergleichbarer Verbund zu Oxidkeramik erzielt. Mit Formulierung C wurde außerdem eine gute Haftwirkung auf Metallen erreicht.

**Tabelle 2**

| **Haftverm ittler** | **Zugfestigkeit [MPa]** | **Zugfestigkeit nach Thermowechselbelastung [MPa]** |
|---|---|---|
| **Lithiumdisilikat-Keramik (e.max CAD; Ivoclar Vivadent)** | | |
| MBP * | 53.7 ± 7.8 | 44.4 ± 7.0 |
| MBS * | 50.6 ± 15.4 | 46.3 ± 8.9 |
| CP ** | 6.1 ± 2.5 | 32.6 ± 10.1 |
| A | 53.0 ± 30.1 | Nicht bestimmt |
| B | 41.3 ± 4.5 | Nicht bestimmt |
| C | 48.7 ± 5.9 | 49.1 ± 7.2 |
| D | 49.8 ± 5.1 | 45.9 ± 9.5 |
| E | 45.7 ± 8.1 | Nicht bestimmt |
| F | 46.1 ± 13.0 | Nicht bestimmt |
| G | 47.5 ± 14.0 | Nicht bestimmt |

| **Leucitverstärkte Silikatkeramik (Empress, Ivoclar Vivadent)** | | |
|---|---|---|
| MBP * | 25.9 ± 8.4 | 32.2 ± 13.6 |
| MBS * | 31.1 ± 11.4 | Nicht bestimmt |
| C | 43.9 ± 3.2 | 53.9 ± 3.8 |

| **Zirkonoxid-Keramik (ZirCAD, Ivoclar Vivadent)** | | |
|---|---|---|
| MBP *** | 49.3 ± 5.2 | 47.1 ± 6.2 |
| CP *** | 40.5 ± 5.8 | Nicht bestimmt |
| ZP *** | 38.0 ± 13.4 | Nicht bestimmt |
| C | 43.3 ± 4.9 | 53.9 ± 3.8 |
| E | 34.1 ± 7.1 | Nicht bestimmt |
| F | 39.4 ± 6.0 | Nicht bestimmt |

| **Aluminiumoxid-Keramik (Al-Cube, Fa. VITA)** | | |
|---|---|---|
| MBP *** | 33.5 ± 13.2 | 11.5 ± 10.9 |
| C | 25.7 ± 4.6 | 10.8 ± 5.8 |

| **Titan (Tritan, 99,5% Titan, Dentaurum)** | | |
|---|---|---|
| MBP*** | 19.6 ± 5.3 | 5.7 ± 3.4 |
| C | 17.9 ± 10.6 | 10.4 ± 8.2 |

| | | |
|---|---|---|
| * Vergleichsbeispiel, Probe mit HF vorgeätzt ** Vergleichsbeispiel, mit Befestigungszement Panavia F2.0 (Fa. Kuraray Europe, Deutschland) *** Vergleichsbeispiel | | |

### Beispiel 3

### Untersuchung der Freisetzung von HF durch ¹⁹F-NMR-Spektroskopie

Die Stabilität der Polyhydrogenfluoridsalze gegen hydrolytische Zersetzung und Freisetzung von HF wurde per ¹⁹F-NMR-Spektroskopie an homogenen Lösungen in H₂O/Ethanol (50/50%) + d₆-Ethanol geprüft. Die spektroskopischen Untersuchungen wurden an Lösungen der Salze NBu₄HF₂ und NBu₄H₂F₃ durchgeführt. Außerdem wurden die Primerzusammensetzungen A (enthaltend NBu₄HF₂, Alkoxysilan-Monomer MPTMS) und C (enthaltend NBu₄H₂F₃, Alkoxysilan-Monomer MPTMS und Phosphorsäureester-Monomer MDP) untersucht. Die Proben wurden (wie in Tabelle 3 vermerkt) teilweise vor der Messung gelagert um eine graduelle HF-Freisetzung durch langsame Hydrolyse sicher ausschließen zu können. An Primer C wurde die spektroskopische Untersuchung vor, während und nach einer 8-wöchigen Stresslagerung bei 50°C zur beschleunigten Alterung vorgenommen.

Die Abwesenheit freier Flusssäure HF in den Primerzusammensetzungen wurde per ¹⁹F-NMR-Spektroskopie durch Abwesenheit des entsprechenden Singulettsignals bei -162.9 bis -165.8 ppm nachgewiesen. Dieses Signal ist auch nach 8-wöchiger Stresslagerung bei 50°C nicht nachweisbar, so dass die Abwesenheit von HF als eine beständige Eigenschaft der erfindungsgemäßen Primerzusammensetzungen angesehen werden kann. Die NMR-Spektren belegen somit die Beständigkeit der erfindungsgemäßen Primerzusammensetzungen enthaltend Polyhydrogenfluoridsalze gegen hydrolytische Zersetzung und Freigabe von HF.

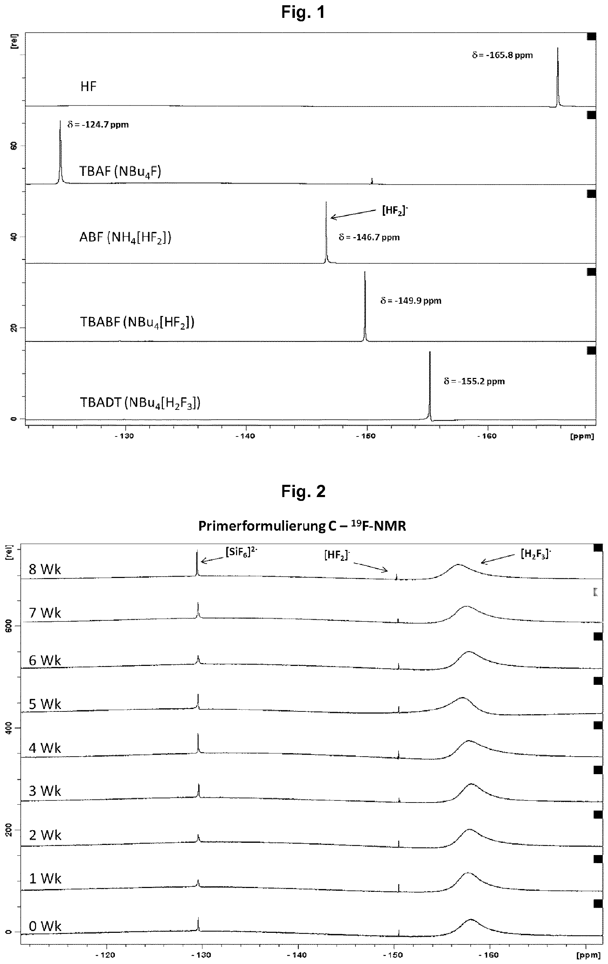

Fig. 1 zeigt ¹⁹F-NMR-Spektren verschiedener Fluoridverbindungen (Fluorwasserstoff, HF; Tetrabutylammoniumfluorid, TBAF, Ammoniumbifluorid, ABF; Tetrabutylammoniumhydrogendifluorid TBABF, Tetrabutylammoniumdihydrogentrifluorid, TBADT). Messung als homogene Lösungen in H₂O/Ethanol (50/50%) + d₆-Ethanol.
Fig. 2 zeigt das ¹⁹F-NMR-Spektrum der Primerzusammensetzung C während Lagerung bei 50°C über 8 Wochen. Messung als homogene Lösungen in H₂O/Ethanol (50/50%) + d₆-Ethanol.

**Tabelle 3**

| **Primer** | **Fluoridquelle** | **Probenalter bei Messung** | **Monomere [Gew.-%]** | **Lösungsmittel [Gew.-%]** | **δ(¹⁹F) ppm (%)** |
|---|---|---|---|---|---|
| - | HF (0.65%, 0.325 M) | frisch hergestellt | - | H₂O/EtOH (50/50%) | -165.8 (singulett) |
| - | NBu₄HF₂ (10%, 0,356 M) | frisch hergestellt | - | H₂O/EtOH (45/45%) | -147.6 (broad) |
| - | NBu₄HF₂ (9.1%, 0,325 M) | 1 Woche bei Raumtemperatur | - | H₂O/EtOH | -149.9 (broad) -129.6 (multi) |
| - | NBu₄H₂F₃ (19,5%, 0,65 M) | frisch hergestellt | - | H₂O/EtOH (40/40%) | -156.6 (broad) -129.9 (multi) |
| - | NBu₄H₂F₃ (10%, 0,325 M) | frisch hergestellt | - | H₂O/EtOH (45/45%) | -155.2 (broad) -130.0 (multi) |
| - | NBu₄H₂F₃ (5%, 0,167 M) | frisch hergestellt | - | H₂O/EtOH (47.5/47.5%) | -154.5 (broad) -130.0 (multi) |
| - | NBu₄H₂F₃ (20%, 0,65 M) | 4 Wochen bei Raumtemperatur | MPTMS (10%) | H₂O/EtOH (35/35%) | -130.4 (broad) -117.1 (multi) |
| A | NBu₄HF₂ (10%, 0,356 M) | frisch hergestellt | MPTMS (1%) | H₂O/EtOH (44.5/44.5%) | -145.6 (broad) -129.6 (multi) -116.7 (multi) |
| C | NBu₄H₂F₃ (10%, 0,325 M) | frisch hergestellt | MPTMS (1%) MDP (1%) | H₂O/EtOH (44.5/44.5%) | -145.6 (broad) -129.6 (multi) -116.7 (multi) |
| C | NBu₄H₂F₃ (10%, 0,325 M) | 8 Wochen bei 50°C | MPTMS (1%) MDP (1%) | H₂O/EtOH (44.5/44.5%) | -145.6 (broad) -129.6 (multi) -116.7 (multi) |

### Beispiel 4

### Rasterelektronenmikroskopie (REM) nasschemisch erodierter Silikatkeramiken

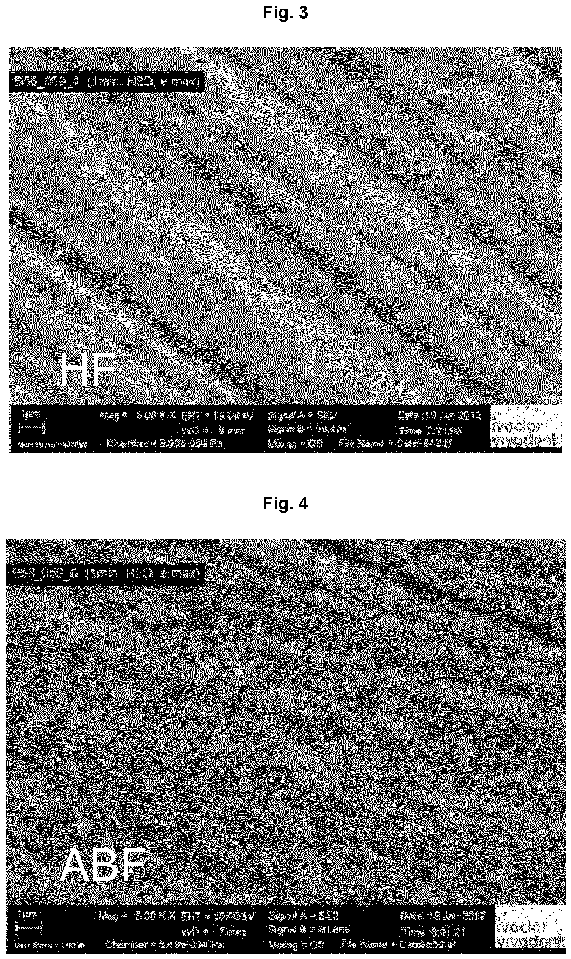

Die Fähigkeit der Polyhydrogenfluoridsalze zur nasschemischen Erosion von Silikatkeramiken wurde per REM-Analytik nachgewiesen (siehe Fig. 3-5). Die Aufnahmen belegen die gewünschte Bildung mikromechanischer Retentionsstellen auf der Keramik bei den dentalmedizinisch üblichen Einwirkzeiten von 1 Minute ohne Agitation. Überraschenderweise war die Bildung mikromechanischer Retentionsstellen mit Polyhydrogenfluoridsalz-Lösungen sogar stärker ausgeprägt als mit einer äquimolaren HF-Lösung im gleichen Lösemittel (H₂O/Ethanol, 0.325 M), was einen weiteren Vorteil der erfindungsgemäßen Primerformulierungen ist.

Fig. 3 zeigt die auf Lithiumdisilikatkeramik (E.max, Ivoclar Vivadent AG) durch Flusssäure, HF (0.325 M; H₂O/Ethanol 50/50 Vol.-%) erzielte nasschemische Erosion nach 1 Minute agitationsfreiem Kontakt und Abspülen mit Wasser.

Fig. 4 zeigt die auf Lithiumdisilikatkeramik (E.max, Ivoclar Vivadent AG) durch Ammo-niumbifluorid, NH₅F₂, ABF (0.325 M; H₂O/Ethanol 50/50 Vol.-%) erzielte nasschemische Erosion nach 1 Minute agitationsfreiem Kontakt und Abspülen mit Wasser.

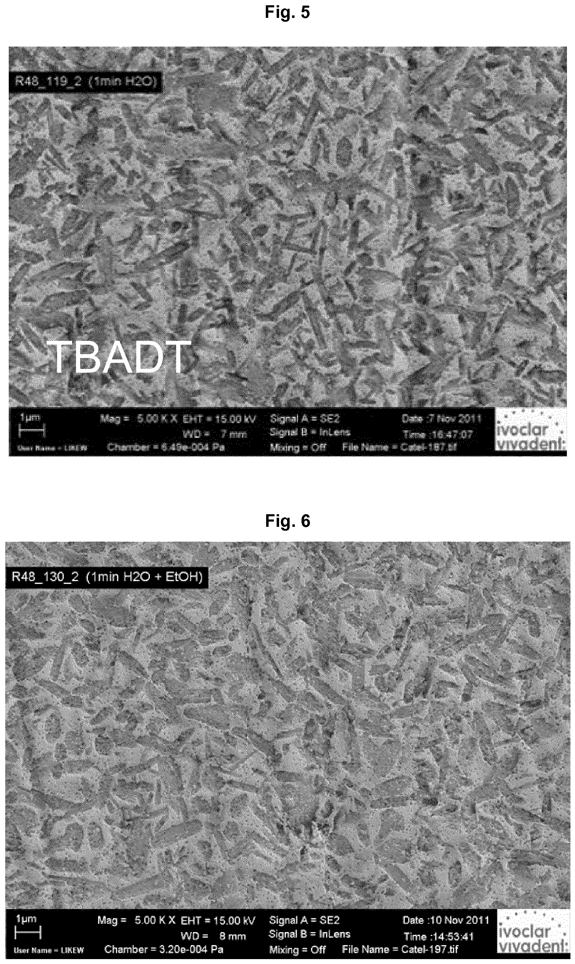

Fig. 5 zeigt die auf Lithiumdisilikatkeramik (E.max, Ivoclar Vivadent AG) durch Tetrabutylammoniumdihydrogentrifluorid NBu₄H₂F₃, TBADT (0.325 M; H₂O/Ethanol 50/50 Vol.-%) erzielte nasschemische Erosion nach 1 Minute agitationsfreiem Kontakt und Abspülen mit Wasser.

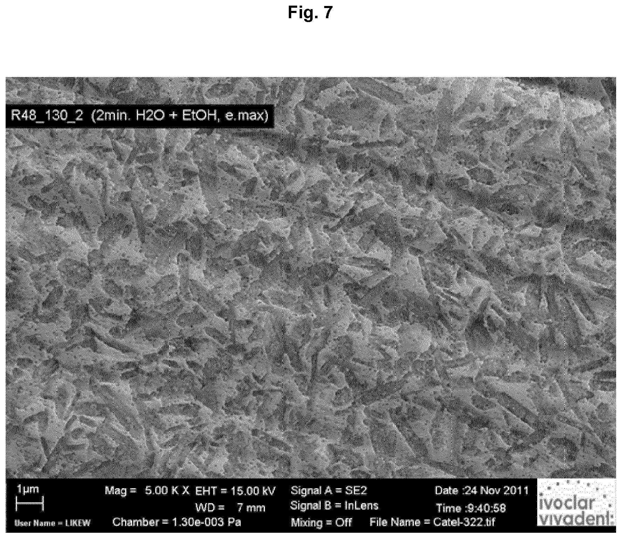

Mit Primerzusammensetzung C wurde die Auswirkung einer verdoppelten Einwirkdauer auf lithiumdisilikatischen Keramiken per REM-Analytik untersucht (Fig. 6 und 7). Das Ätzmuster nach 1 Minute Kontaktdauer ist nicht von dem nach 2 Minuten Kontaktdauer zu unterscheiden. Weder waren die für überätzte Lithiumdisilikat-Keramiken kennzeichnenden weißlichen Ablagerungen aus freigelegten Lithiumdisilikat-Kristalliten auf der Keramikoberfläche zu beobachten, noch werden nicht in die amorphe Phase eingebundene Kristallite in den REM-Aufnahmen sichtbar. Solche lose gebundenen Kristallite bilden im Haftverbund zwischen Keramik und Befestigungsmaterial Sollbruchstellen und können durch ihre Anwesenheit die Haftwirkung beträchtlich schwächen. Es ist daher vorteilhaft für den Anwender, das Überätzen einer Keramik sicher vermeiden zu können. Die geringe Neigung der Primerzusammensetzungen zum Überätzen der Keramik bei unabsichtlich verlängertem Kontakt stellt also einen beträchtlichen Vorteil der erfindungsgemäßen Primerzusammensetzung gegenüber der klassischen HF-Ätzung dar.

Fig. 6 zeigt die auf Lithiumdisilikatkeramik (E.max, Ivoclar Vivadent AG) durch Primerformulierung C (enthaltend Tetrabutylammoniumdihydrogentrifluorid, NBu₄H₂F₃, TBADT; 0.325 M; H₂O/Ethanol 50/50 Vol.-%) erzielte nasschemische Erosion nach 1 Minute agitationsfreiem Kontakt und Abspülen mit Wasser.

Fig. 7 zeigt die auf Lithiumdisilikatkeramik (E.max, Ivoclar Vivadent AG) durch Primerformulierung C (enthaltend Tetrabutylammoniumdihydrogentrifluorid, NBu₄H₂F₃, TBADT; 0.325 M; H₂O/Ethanol 50/50 Vol.-%) erzielte nasschemische Erosion nach 2 Minute agitationsfreiem Kontakt und Abspülen mit Wasser.

## Patentansprüche

1. Dentale Primerzusammensetzung, die
(1) mindestens ein Alkoxysilan-Monomer der allgemeinen Formel (I)
R¹ₙSi(OR²)₄₋ₙ (I),
worin
R¹ für einen organischen Rest steht, der mindestens eine ethylenisch ungesättigte, polymerisierbare Gruppe aufweist,
R² für einen C₁- bis C₈-alkylorganischen Rest steht und
n 1,2 oder 3 ist,
wobei die Reste R¹ und R² jeweils gleich oder unterschiedlich sein können;
(2) mindestens ein Polyhydrogenfluoridsalz der allgemeinen Formel (II)
(R⁹)⁺(Hₓ₋₁Fₓ)⁻_{z} (II),
worin
R⁹ für ein Metallkation aus der Reihe der Alkali-, Erdalkali oder Übergangsmetalle oder für ein Ammoniumion der Formel (R⁵)(R⁶)(R⁷)(R⁸)N⁺ steht, in der R⁵, R⁶, R⁷ und R⁸ jeweils unabhängig voneinander für H oder C₁- bis C₂₆-Alkyl-, C₃- bis C₂₆-Alkenyl- oder C₆-C₂₆-Arylreste stehen, wobei R⁵, R⁶, R⁷ und R⁸ gleich oder unterschiedlich sein können, und wobei zwei dieser Reste miteinander verbunden sein können, um zusammen mit dem Stickstoffatom einen Heterocyclus zu bilden und wobei drei der Reste und das Stickstoffatom zusammen ein Pyridiniumion bilden können,
x eine ganze Zahl von 2 bis 5, vorzugsweise 2 bis 4, insbesondere 3 ist,
z der Wertigkeit des kationischen Restes R⁹ entspricht,
(3) organisches Lösungsmittel, und
(4) Wasser enthält.

2. Zusammensetzung nach Anspruch 1 in der mindestens eine oder alle Variablen eine der folgenden Bedeutungen haben:
Formel (I):
R¹ = ein Rest mit der folgenden Formel: 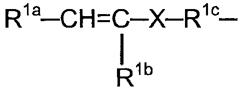 in der
R^{1a} = H oder Phenyl, vorzugsweise H ist;
R^{1b} = H oder Methyl, vorzugsweise Methyl ist;
R^{1c} = entfällt oder C₁-C₁₆-Alkyl, vorzugsweise C₁-C₃-Alkyl ist;
X = entfällt oder -CO-O- oder -CO-NH-, vorzugsweise -CO-O- oder -CO-NH- ist, wobei X entfällt, wenn R^{1c} entfällt;
R² = H oder C₁- bis C₂-Alkyl und
n = 1 oder 2,
wobei die Reste R¹ und R² jeweils gleich oder unterschiedlich sein können und vorzugsweise gleich sind;
Formel (II)
x = eine ganze Zahl von 2 bis 4, vorzugsweise 3,
z = 1,
R⁹ = ein Ammoniumion der Formel (R⁵)(R⁶)(R⁷)(R⁸)N⁺, in der R⁵, R⁶, R⁷ und R⁸ unabhängig voneinander H, n- oder i-C₁-C₄-Alkyl bedeuten, wobei R⁵, R⁶, R⁷ und R⁸ vorzugsweise gleich sind, bevorzugt sind R⁵ = R⁶ = R⁷ = R⁸ = Butyl.

3. Zusammensetzung nach einem der Ansprüche 1 bis 2, die als Lösungsmittel einen Alkohol, ein Keton, einen Ester, Methanol, Ethanol, n-Propanol, i-Propanol, t-Butanol, Ethylacetat, Aceton, Methylethylketon oder eine Mischung davon enthält.

4. Zusammensetzung nach einem der Ansprüche 1 bis 3, die keine freie Flusssäure (HF) enthält.

5. Zusammensetzung nach einem der Ansprüche 1 bis 4, die zusätzlich mindestens ein Phosphorsäure- oder Pyrophosphorsäureester-Monomer der Formel (III) enthält:
O=P(OR³)ₘ(OR⁴)₃₋ₘ (III),
worin
R³ für einen organischen Rest steht, der mindestens eine ethylenisch ungesättigte, polymerisierbare Gruppe aufweist,
R⁴ für H, SiR₃, P(=O)(OR¹⁴)₂ oder C₁- bis C₁₆-Alkyl steht, wobei R¹⁴ für H, SiR'₃ oder C₁- bis C₁₆-Alkyl steht und wobei R und R' unabhängig voneinander jeweils C₁ bis C₄-Alkyl sind, und
m 1 oder 2 ist,
wobei die Reste R³ und R⁴ jeweils gleich oder unterschiedlich sein können, und/oder
mindestens ein Phosphonsäure- oder Polyphosphonsäure-Monomer der allgemeinen Formel (IIIa) enthält:
R¹¹[P(=O)(OR¹⁰)₂]ₙ (IIIa)
worin
R¹¹ für einen organischen Rest steht, der mindestens eine ethylenisch ungesättigte, polymerisierbare Gruppe aufweist,
R¹⁰ für einen Rest ausgewählt aus H, SiR"₃ oder C₁- bis C₁₆-Alkyl steht, wobei R" C₁ bis C₄-Alkyl ist,
n 1, 2, 3 oder 4 ist.

6. Zusammensetzung nach einem der Ansprüche 1 bis 5, die zusätzlich mindestens ein Hilfsmittel enthält, das aus Netzmitteln, Detergenzien, nichtionische, anionische und/oder kationische Tensiden, Entschäumern, Stabilisatoren, antimikrobiellen Zusätzen, Duftstoffen, Farbstoffen, Konservierungsmitteln, polymeren Verdickungsmitteln, Thixotropieagenzien und Rheologiemodifizierern ausgewählt ist.

7. Zusammensetzung nach einem der Ansprüche 1 bis 6, die zusätzlich ein Alkoxysilan der allgemeinen Formel (la) enthält,
(OR¹³)₃SiR¹²Si(OR¹³)₃ (Ia),
worin
R¹² für C₁-C₁₂-Alkylen, C₁-C₁₂-Heteroalkylen oder C₆-C₁₂-Arylen steht,
R¹³ unabhängig jeweils für H oder C₄- bis C₈-Alkyl steht,
wobei die Reste R¹³ jeweils gleich oder unterschiedlich sein können.

8. Zusammensetzung nach einem der Ansprüche 1 bis 7, die
(1) 0,05 bis 25,0 Gew.-%, bevorzugt 0,2 bis 10,0 Gew.-% und besonders bevorzugt 0,5 bis 5,0 Gew.-% Alkoxysilan-Monomer (I);
(2) 1,0 bis 25,0 Gew.-%, bevorzugt 2,0 bis 15,0 Gew.-% und besonders bevorzugt 5,0 bis 10,0 Gew.-% Polyhydrogenfluoridsalz (II);
(3) 25 bis 98,5 Gew.-%, bevorzugt 35 bis 75 Gew.-% und besonders bevorzugt 45 bis 55 Gew.-% organisches Lösungsmittel;
(4) 25 bis 98,5 Gew.-%, bevorzugt 35 bis 75 Gew.-% und besonders bevorzugt 40 bis 75 Gew.-% Wasser enthält,
jeweils bezogen auf das Gesamtgewicht der Zusammensetzung.

9. Zusammensetzung nach Anspruch 8, die zusätzlich
- 0,005 bis 2,5 Gew.-%, bevorzugt 0,02 bis 1,0 Gew.-% und besonders bevorzugt 0,05 bis 0,5 Gew.-% des Alkoxysilans (Ia); und/oder
- 0,05 bis 25,0 Gew.-%, bevorzugt 0,2 bis 10,0 Gew.-% und besonders bevorzugt 0,5 bis 5,0 Gew.-% Phosphorsäureester-Monomer (III) und/oder
- 0,05 bis 25,0 Gew.-%, bevorzugt 0,2 bis 10,0 Gew.-% und besonders bevorzugt 0,5 bis 5,0 Gew.-% Phosphonsäure-Monomer (IIIa); und/oder
- 0,001 bis 10%, bevorzugt 0,1 bis 7,5%, besonders bevorzugt 1,0 bis 5% Hilfsmittel enthält,
jeweils bezogen auf das Gesamtgewicht der Zusammensetzung.

10. Zusammensetzung gemäß einem der Ansprüche 1 bis 9 zur Verwendung in der Zahnheilkunde.

11. Verwendung einer Zusammensetzung gemäß einem der Ansprüche 1 bis 9 zur Oberflächenbehandlung von metallischen oder keramischen Dentalrestaurationen oder Dentalmaterialien.

12. Verwendung nach Anspruch 11 zur Oberflächenbehandlung einer Dentalrestauration oder eines Dentalmaterials auf Basis von Silikatkeramik.

13. Verwendung einer Zusammensetzung gemäß einem der Ansprüche 1 bis 9 als Haftvermittler.

14. Dentalrestauration, **dadurch gekennzeichnet, dass** zumindest ein Teil ihrer Oberfläche mit einer Zusammensetzung gemäß einem der Ansprüche 1 bis 9 behandelt ist.

15. Dentalrestauration nach Anspruch 14, die eine Oberfläche aus silikatischer Keramik, Feldspat, Quarz, Leucit- oder Lithiumdisilikat-basierter Keramik hat.

## Claims

1. Dental primer composition comprising
1. at least one alkoxysilane monomer of the general formula (I)
R¹ₙSi(OR²)₄₋ₙ (I),
in which
R¹ stands for an organic residue that possesses at least one ethylenically unsaturated, polymerisable group,
R² stands for a C₁-C₈ alkyl organic residue and
n is 1, 2 or 3,
wherein the residues R¹ and R² can each be the same or different;
(2) at least one poly(hydrogen fluoride) salt of the general formula (II)
(R⁹)⁺(Hₓ₋₁Fₓ)⁻_{z} (II),
in which
R⁹ stands for a metal cation from the series of the alkali, alkaline earth or transition metals or for an ammonium ion of the formula (R⁵)(R⁶)(R⁷)(R⁸)N⁺, in which R⁵, R⁶, R⁷ and R⁸ each independently of each other stand for H or C₁ to C₂₆ alkyl, C₃ to C₂₆ alkenyl or C₆-C₂₆ aryl residues, wherein R⁵, R⁶, R⁷ and R⁸ can be the same or different, and wherein two of these residues can be linked together to form a heterocycle with the nitrogen atom and wherein three of the residues and the nitrogen atom can together form a pyridinium ion,
x is an integer from 2 to 5, preferably 2 to 4, in particular 3,
z corresponds to the valence of the cationic residue R⁹,
(3) organic solvent, and
(4) water.

2. Composition according to claim 1, in which at least one or all variables have one of the following meanings:
Formula (I):
R¹ = a residue with the following formula: 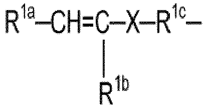 in which
R^{1a} = H or phenyl, preferably H;
R^{1b} = H or methyl, preferably methyl;
R^{1c} = is absent or is C₁-C₁₆ alkyl, preferably C₁-C₃ alkyl;
X is absent or is -CO-O- or -CO-NH-, preferably -CO-O- or -CO-NH-, wherein X is absent if R^{1c} is absent;
R² = H or C₁ to C₂ alkyl and
n=1 or 2,
wherein the residues R¹ and R² can each be the same or different and are preferably the same;
Formula (II):
x = an integer from 2 to 4, preferably 3,
z=1,
R⁹ = an ammonium ion of the formula (R⁵)(R⁶)(R⁷)(R⁸)N⁺, in which R⁵, R⁶, R⁷ and R⁸ independently of each other stand for H, *n*- or *i*-C₁-C₄ alkyl, C₃ to C₂₆ alkenyl or C₆-C₂₆ aryl residues, wherein R⁵, R⁶, R⁷ and R⁸ are preferably the same, preferably R⁵ = R⁶ = R⁷ = R⁸ = butyl.

3. Composition according to one of claims 1 to 2, comprising as the solvent an alcohol, a ketone, an ester, methanol, ethanol, *n*-propanol, *i*-propanol, *t*-butanol, ethyl acetate, acetone, methyl ethyl ketone or a mixture thereof.

4. Composition according to one of claims 1 to 3, which contains no free hydrofluoric acid (HF).

5. Composition according to one of claims 1 to 4, which additionally comprises at least one phosphoric acid ester monomer or pyrophosphoric acid ester monomer of Formula (III):
O=P(OR³)ₘ(OR⁴)₃₋ₘ (III),
in which
R³ stands for an organic residue that possesses at least one ethylenically unsaturated, polymerisable group,
R⁴ stands for H, SiR₃, P(=O)(OR¹⁴)₂ or C₁ to C₁₆ alkyl, wherein R¹⁴ stands for H, SiR'₃ or C₁ to C₁₆ alkyl and wherein R and R' are each independently of each other C₁ to C₄ alkyl, and
m is 1 or 2,
wherein the residues R³ and R⁴ can each be the same or different,
and/or
comprises at least one phosphonic acid or polyphosphonic acid monomer of the general formula (IIIa):
R¹¹[P(=O)(OR¹⁰)₂]ₙ (IIIa)
in which
R¹¹ stands for an organic residue that possesses at least one ethylenically unsaturated, polymerisable group,
R¹⁰ stands for a residue selected from H, SiR"₃ or C₁ to C₁₆ alkyl, wherein R" is C₁ to C₄ alkyl,
n is 1, 2, 3 or 4.

6. Composition according to one of claims 1 to 5, which additionally comprises at least one auxiliary agent that is selected from wetting agents, detergents, non-ionic, anionic and/or cationic surfactants, antifoaming agents, stabilisers, antimicrobial additives, fragrances, colorants, preservatives, polymeric thickeners, thixotropic agents and rheology modifiers.

7. Composition according to one of claims 1 to 6, additionally comprising an alkoxysilane of the general formula (Ia),
(OR¹³)₃SiR¹²Si(OR¹³)₃ (Ia),
in which
R¹² stands for C₁-C₁₂ alkylene, C₁-C₁₂ heteroalkylene or C₆-C₁₂ arylene,
R¹³ each independently stands for H or C₁ to C₈ alkyl,
wherein the residues R¹³ can each be the same or different.

8. Composition according to one of claims 1 to 7, comprising
(1) 0.05 to 25.0 wt %, preferably 0.2 to 10.0 wt % and particularly preferably 0.5 to 5.0 wt % of alkoxysilane monomer (I);
(2) 1.0 to 25.0 wt %, preferably 2.0 to 15.0 wt % and particularly preferably 5.0 to 10.0 wt % of poly(hydrogen fluoride) salt (II);
(3) 25 to 98.5 wt %, preferably 35 to 75 wt % and particularly preferably 45 to 55 wt % of organic solvent;
(4) 25 to 98.5 wt % preferably 35 to 75 wt % and particularly preferably 40 to 75 wt % of water;
each based on the total weight of the composition.

9. Composition according to claim 8, additionally comprising
- 0.005 to 2.5 wt %, preferably 0.02 to 1.0 wt % and particularly preferably 0.05 to 0.5 wt % of the alkoxysilane (Ia); and/or
- 0.05 to 25.0 wt %, preferably 0.2 to 10.0 wt % and particularly preferably 0.5 to 5.0 wt % of phosphoric acid ester monomer (III) and/or
- 0.05 to 25.0 wt %, preferably 0.2 to 10.0 wt % and particularly preferably 0.5 to 5.0 wt % of phosphonic acid monomer (IIIa) and/or
- 0.001 to 10%, preferably 0.1 to 7.5 wt %, particularly preferably 1.0 to 5% of auxiliaries,
each based on the total weight of the composition.

10. Composition according to one of claims 1 to 9 for use in dentistry.

11. Use of a composition according to one of claims 1 to 9 for the surface treatment of metallic or ceramic dental restorations or dental materials.

12. Use according to claim 11 for the surface treatment of a dental restoration or of a dental material based on silicate ceramic.

13. Use of a composition according to one of claims 1 to 9 as an adhesion promotor.

14. Dental restoration, **characterised in that** at least a part of its surface is treated with a composition according to one of claims 1 to 9.

15. Dental restoration according to claim 14 which has a surface of silicate ceramic, feldspar, quartz, leucite- or lithium disilicate-based ceramic.

## Revendications

1. Composition de primaire dentaire, qui contient :
1) au moins un monomère alcoxy-silane de formule générale (I) :
R¹ₙSi(OR²)₄₋ₙ (I)
dans laquelle
- R¹ représente un reste organique comportant au moins un groupe polymérisable à insaturation éthylénique,
- R² représente un reste organique alkyle en C₁-C₈,
- et l'indice n vaut 1, 2 ou 3,
étant entendu que les restes représentés par chacun des symboles R¹ et R² peuvent être identiques ou différents ;
2) au moins un sel polyfluorhydrate de formule générale (II) :
(R⁹)⁺(Hₓ₋₁Fₓ)⁻_{z} (II)
dans laquelle
- R⁹ représente un cation métallique de la série des métaux alcalins, des métaux alcalino-terreux ou des métaux de transition, ou un ion ammonium de formule (R⁵)(R⁶)(R⁷)(R⁸)N⁺ dans laquelle R⁵, R⁶, R⁷ et R⁸ représentent chacun, indépendamment les uns des autres, un atome d'hydrogène ou un groupe alkyle en C₁-C₂₆, alcényle en C₃-C₂₆ ou aryle en C₆-C₂₆, étant entendu que les restes symbolisés par R⁵, R⁶, R⁷ et R⁸ peuvent être identiques ou différents, que deux de ces restes peuvent être raccordés l'un à l'autre pour former un hétérocycle avec l'atome d'azote, et que trois de ces restes et l'atome d'azote peuvent former ensemble un ion pyridinium,
- l'indice x est un nombre entier valant de 2 à 5, de préférence de 2 à 4 et en particulier 3,
- et l'indice z correspond à la valence du reste cationique R⁹ ;
3) un solvant organique ;
4) et de l'eau.

2. Composition conforme à la revendication 1, dans laquelle au moins l'un des symboles a, ou tous les symboles ont, l'une des significations suivantes :
dans la formule (I) :
- R¹ représente un reste de formule suivante : 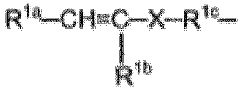 dans laquelle
- R^{1a} représente un atome d'hydrogène ou un groupe phényle,
et de préférence un atome d'hydrogène,
- R^{1b} représente un atome d'hydrogène ou un groupe méthyle,
et de préférence un groupe méthyle,
- R^{1c} ne représente rien ou représente un groupe alkyle en C₁-C₁₆, et de préférence un groupe alkyle en C₁-C₃,
- et X ne représente rien ou représente un raccord symbolisé par -CO-O- ou -CO-NH-, et de préférence un raccord -CO-O- ou -CO-NH-, étant entendu que X ne représente rien si R^{1c} ne représente rien,
- R² représente un atome d'hydrogène ou un groupe alkyle en C₁-C₂,
- et l'indice n vaut 1 ou 2,
étant entendu que les restes représentés par chacun des symboles R¹ et R² peuvent être identiques ou différents, et sont de préférence identiques ;
et dans la formule (II) :
- l'indice x est un nombre entier valant de 2 à 4, et de préférence 3,
- l'indice z vaut 1,
- et R⁹ représente un ion ammonium de formule (R⁵)(R⁶)(R⁷)(R⁸)N⁺ dans laquelle R⁵, R⁶, R⁷ et R⁸ représentent chacun, indépendamment les uns des autres, un atome d'hydrogène ou un groupe n-alkyle en C₁-C₄ ou i-alkyle en C₁-C₄, étant entendu que les restes symbolisés par R⁵, R⁶, R⁷ et R⁸ sont de préférence identiques et que, mieux encore, R⁵, R⁶, R⁷ et R⁸ représentent chacun un groupe butyle.

3. Composition conforme à l'une des revendications 1 et 2, qui contient, en tant que solvant, un alcool, une cétone, un ester, du méthanol, de l'éthanol, du n-propanol, de l'isopropanol, du tertio-butanol, de l'acétate d'éthyle, de l'acétone, de la méthyl-éthyl-cétone ou un mélange de tels corps.

4. Composition conforme à l'une des revendications 1 à 3, qui ne contient pas d'acide fluorhydrique libre (HF).

5. Composition conforme à l'une des revendications 1 à 4, qui contient en outre au moins un monomère ester d'acide phosphorique ou pyrophosphorique de formule (III) :
O=P(OR³)ₘ(OR⁴)₃₋ₘ (III)
dans laquelle
- R³ représente un reste organique comportant au moins un groupe polymérisable à insaturation éthylénique,
- R⁴ représente un atome d'hydrogène, un groupe alkyle en C₁-C₁₆, ou un reste de formule SiR₃ ou P(=O)(OR¹⁴)₂, où R¹⁴ représente un atome d'hydrogène, un groupe alkyle en C₁-C₁₆ ou un reste de formule SiR'₃, étant entendu que R et R' représentent chacun, indépendamment l'un de l'autre, un groupe alkyle en C₁-C₄,
- et l'indice m vaut 1 ou 2,
étant entendu que les restes représentés par chacun des symboles R³ et R⁴ peuvent être identiques ou différents ;
et/ou au moins un monomère acide phosphonique ou polyphosphonique de formule générale (IIIa) :
R¹¹[P(=O)(OR¹⁰)₂]ₙ (IIIa)
dans laquelle
- R¹¹ représente un reste organique comportant au moins un groupe polymérisable à insaturation éthylénique,
- R¹⁰ représente un reste choisi parmi un atome d'hydrogène, un groupe alkyle en C₁-C₁₆ et un reste de formule SiR"₃, étant entendu que R" représente un groupe alkyle en C₁-C₄,
- et l'indice n vaut 1, 2, 3 ou 4.

6. Composition conforme à l'une des revendications 1 à 5, qui contient en outre au moins un adjuvant choisi parmi les suivants : agents mouillants, détergents, tensioactifs non-ioniques, anioniques et/ou cationiques, agents anti-mousse, stabilisants, additifs anti-microbiens, parfums, colorants, conservateurs, épaississants polymères, agents de thixotropie, et modificateurs de rhéologie.

7. Composition conforme à l'une des revendications 1 à 6, qui contient en outre un alcoxy-silane de formule générale (Ia) :
(OR¹³)₃SiR¹²Si(OR¹³)₃ (Ia)
dans laquelle
- R¹² représente un groupe alcanediyle en C₁-C₁₂, hétéroalcanediyle en C₁-C₁₂ ou arènediyle en C₆-C₁₂,
- et les symboles R¹³ représentent chacun, indépendamment l'un de l'autre, un groupe alkyle en C₁-C₈,
étant entendu que les restes représentés par chacun des symboles R¹³ peuvent être identiques ou différents.

8. Composition conforme à l'une des revendications 1 à 7, qui contient :
1) de 0,05 à 25,0 % en poids, de préférence de 0,2 à 10,0 % en poids et mieux encore de 0,5 à 5,0 % en poids de monomère alcoxy-silane de formule (I),
2) de 1,0 à 25,0 % en poids, de préférence de 2,0 à 15,0 % en poids et mieux encore de 5,0 à 10,0 % en poids de sel polyfluorhydrate de formule (II),
3) de 25 à 98,5 % en poids, de préférence de 35 à 75 % en poids et mieux encore de 45 à 55 % en poids de solvant organique,
4) et de 25 à 98,5 % en poids, de préférence de 35 à 75 % en poids et mieux encore de 40 à 75 % en poids d'eau,
par rapport au poids total de la composition, pour chaque composant.

9. Composition conforme à la revendication 8, qui contient en outre :
- de 0,005 à 2,5 % en poids, de préférence de 0,02 à 1,0 % en poids et mieux encore de 0,05 à 0,5 % en poids d'alcoxy-silane de formule (la),
- et/ou de 0,05 à 25,0 % en poids, de préférence de 0,2 à 10,0 % en poids et mieux encore de 0,5 à 5,0 % en poids de monomère de type ester d'acide phosphorique de formule (III),
- et/ou de 0,05 à 25,0 % en poids, de préférence de 0,2 à 10,0 % en poids et mieux encore de 0,5 à 5,0 % en poids de monomère de type acide phosphonique de formule (IIIa),
- et/ou de 0,001 à 10 % en poids, de préférence de 0,1 à 7,5 % en poids et mieux encore de 1,0 à 5 % en poids d'adjuvant(s),
par rapport au poids total de la composition, pour chaque composant.

10. Composition conforme à l'une des revendications 1 à 9, pour utilisation en médecine dentaire.

11. Utilisation d'une composition conforme à l'une des revendications 1 à 9 pour le traitement de surface de restaurations dentaires ou matériaux dentaires en métal ou en céramique.

12. Utilisation conforme à la revendication 11 pour le traitement de surface d'une restauration dentaire ou d'un matériau dentaire à base de céramique silicatée.

13. Utilisation d'une composition conforme à l'une des revendications 1 à 9 en tant qu'agent adhésif.

14. Restauration dentaire, **caractérisée en ce qu'**au moins une partie de sa surface a été traitée avec une composition conforme à l'une des revendications 1 à 9.

15. Restauration dentaire conforme à la revendication 14, qui possède une surface en céramique silicatée, en feldspath, en quartz, ou en céramique à base de leucite ou de disilicate de lithium.
